(19) Europäisches Patentamt
European Patent Office
Office européen des brevets

(11) Publication number: **0 029 587**
**B1**

# (12) EUROPEAN PATENT SPECIFICATION

(45) Date of publication of patent specification: **13.06.84**

(51) Int. Cl.³: **C 07 D 337/14,**
A 61 K 31/38

(21) Application number: **80107205.9**

(22) Date of filing: **20.11.80**

(54) **3-Hydroxymethyldibenzo(b,f)thiepins and dibenzo(b,f)thiepin-3-carboxaldehydes as prostaglandin antagonists, process for their preparation and pharmaceutical compositions.**

(30) Priority: **27.11.79 US 97755**
**27.11.79 US 97759**

(43) Date of publication of application:
**03.06.81 Bulletin 81/22**

(45) Publication of the grant of the patent:
**13.06.84 Bulletin 84/24**

(84) Designated Contracting States:
**AT BE CH DE FR GB IT LI LU NL SE**

(56) References cited:
**EP - A - 0 000 978**
**EP - A - 0 011 067**
**DE - A - 2 754 561**

(73) Proprietor: **MERCK SHARP & DOHME (I.A.) CORP.**
**126 East Lincoln Avenue**
**Rahway, New Jersey 07065 (US)**

(72) Inventor: **Hamel, Pierre A.**
**1770 Vienne**
**Laval Quebec (CA)**
Inventor: **Rokach, Joshua A.**
**416 Canterbury Place**
**Chomedey-Laval Quebec (CA)**

(74) Representative: **Blum, Rudolf Emil Ernst et al,**
**c/o E. Blum & Co Patentanwälte Vorderberg 11**
**CH-8044 Zürich (CH)**

**EP 0 029 587 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Summary of the Invention

This invention relates to prostaglandin antagonists useful in treating a variety of conditions, such as allergic asthma where excessive contractile activity of prostaglandins and prostaglandin biosynthetic intermediates occur.

The new inventive prostaglandin analogs are dibenzo[b,f]thiepins having a carboxaldehyde substituent in the 3-position or a hydroxyalkyl substituent in the 3-position. The inventive prostaglandin antagonists, accordingly, have the following formula

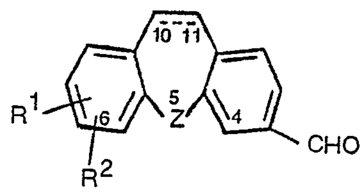

Ia

or Ib

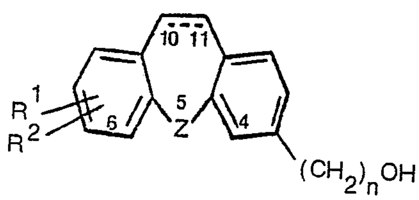

Ib

wherein

Z is thio, sulfinyl, or sulfonyl,

$R^1$ and $R^2$ are independently from each other hydrogen, halogen, amino, alkylamino having 1—4 carbon atoms in the alkyl group, dialkylamino having 1—4 carbon atoms in each of the alkyl groups, alkyl having 1—4 carbon atoms, $C_1$—$C_4$-hydroxyalkyl, $C_1$—$C_4$-phenylalkyl wherein the phenyl group is optionally substituted by halogen, an amino, hydroxy or $C_1$—$C_4$-alkyl group, alkanoyl having 1—4 carbon atoms, hydroxy, thiol, alkoxy having 1—4 carbon atoms, alkylthio, alkylsulfinyl or alkylsulfonyl, wherein in each of said sulphur containing groups the alkyl group has 1—4 carbon atoms, trifluoromethyl, trifluoromethylthio, cyano or nitro n is an integer of from 1—4 and the dotted line indicates either an olefinic bond or saturation at the 10-, 11-position.

In the DE—AI—2,754,561 there are described among other compounds also substances which are structurally related to the inventive compounds of formulae Ia and Ib, in which, however, the substituent in the one benzene nucleus is not an aldehyde group or a hydroxyalkyl group having 1—4 carbon atoms but a propionic acid group having the structure

$CH_3$
|
—CH—COOH

or they are lower alkyl esters or acid amides of said propionic acid derivatives. Said prior art compounds, however, differ as to their pharmaceutical activity from the new inventive compounds. As there can be seen from page 23, last paragraph, until page 28, first paragraph of said German Offenlegungsschrift, said prior art compounds have an anti-inflammatory activity. An anti-inflammatory activity is a typical pharmaceutical activity of a prostaglandin and, accordingly, said prior art compounds are not prostaglandin antagonists.

In the EP—AI—0000978 there are described compounds which are prostaglandin antagonists and, accordingly, have a pharmaceutical activity which is similar to the pharmaceutical activity of the inventive compounds. Said prior art compounds, however, differ from the inventive compounds clearly as to their chemical structure because they have a keto group in the 11-position of the 7-membered ring and furthermore also with regard to the substituent in the 3-position of the benzene nucleus which group, among others, can also be a carboxylic acid group of the formula

$$—(CH_2)_n—COOH$$

wherein n is 0 or an integer from 1—4 or said compounds are derivatives of said acids like alkyl esters or acid amides. The compounds of said European patent publication, accordingly, cannot be regarded as

being structurally related to the new inventive compounds.

The new inventive compounds are used as agents which act as prostaglandin antagonists and they, therefore, offer new approaches to therapy in a number of disease states. The new inventive dibenzo[b,f] thiepin derivatives antagonize the actions of contractile prostaglandins, such as $PGF_{2\alpha}$, $PGG_2$, $PGH_2$, and $TXA_2$. The prostaglandins $PGF_{2\alpha}$, PGG, and $PGH_2$, for example, are potent contractants of bronchial muscle. Indeed human asthmatics have been shown to be especially sensitive to the bronchial constricting action of $PFG_{2\alpha}$.

In addition to the involvement of contractile prostaglandins in chronic obstructive lung disease (or asthma), prostaglandins are known to play a role in other allergic conditions, as well as inflammation, diarrhea, hypertension, angina, platelet aggregation, cerebral spasm, premature abortion, and dismenorrhea.

In addition to the prostaglandin antagonist actions, the dibenzo[b,f]thiepins of this invention are antagonists of slow reacting substance of anaphylaxis (SRS—A). This contractile substance is released in the lung tissue in allergic asthma, and antagonism of its actions contributes to alleviation of this disease.

A further object of the present invention, accordingly, is a composition for treating the contractile activity of prostaglandins which comprises as active ingredient a compound of formula Ia

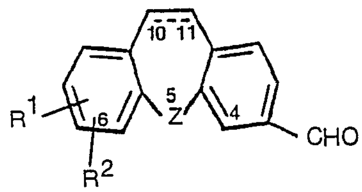

Ia

or Ib

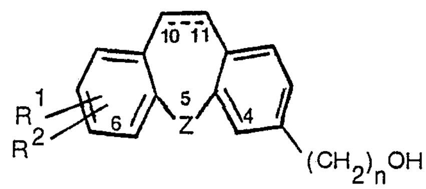

Ib

wherein

Z, $R^1$, $R^2$, n and the dotted line have the same meaning as defined above.

As used herein, the term halogen (or halo) includes chlorine, bromine, iodine, and fluorine. Unless otherwise specifically stated, the terms loweralkyl and loweralkoxy include straight and branched chain alkyl and alkoxy groups having 1—4 carbon atoms in the alkyl or alkoxy moiety such as, for example, methyl, ethyl, n-propyl, isopropyl, n-butyl, methoxy, ethoxy, n-propoxy, and isobutoxy. The term lower-alkanoyl includes straight or branched chain alkanoyl groups having 1—4 carbon atoms in the alkanoyl moiety such as, for example, formyl, acetyl, propanoyl, and isobutyryl. The term phen-$C_1$—$C_4$-alkyl means straight or branched chain $C_1$—$C_4$-alkyl radicals having one of the hydrogens replaced by a phenyl or substituted phenyl group meaning phenyl, halophenyl such as chloro, bromo, iodo, and fluorophenyl, nitrophenyl, aminophenyl, hydroxyphenyl, loweralkylphenyl.

A further object of the present invention is a process for preparing a compound of formula Ia

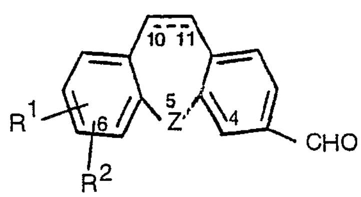

Ia

wherein

Z, $R^1$, $R^2$ and the dotted line have the same meaning as outlined above, which process is characterized in that a corresponding 3-cyano compound having the formula

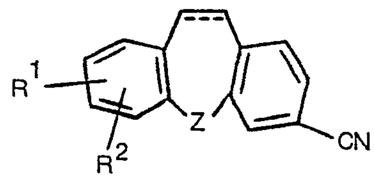

wherein
$R^1$, $R^2$, Z and the dotted line have the same meaning as in formula Ia is reduced and hydrolized.

The present invention, furthermore, also concerns a process for the preparation of a compound of formula Ib

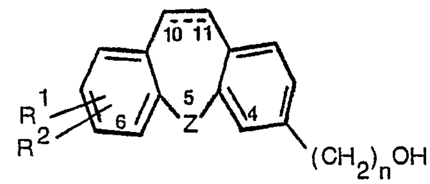

Ib

or Ia

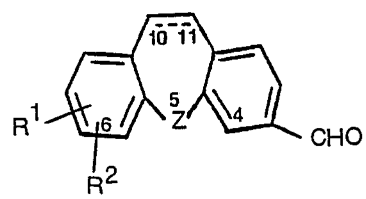

Ia

wherein
Z, $R^1$, $R^2$, n and the dotted line have the same meaning as outlined above, which process is characterized in that a carboxylic acid of formula

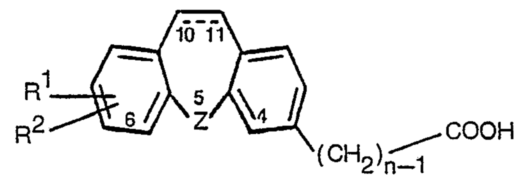

wherein
$R^1$, $R^2$, Z and n are as defined in formula Ib, or a lower alkyl ester thereof is reduced to form the corresponding hydroxyalkyl substituted compound of formula Ib, which compound of formula Ib is isolated and if in formula Ib n is 1, the 3-hydroxymethyl compound is optionally oxidized yielding the aldehyde of formula Ia.

An appropriately substituted dibenzo [b,f]thiepin-3-carboxylic acid or a loweralkyl ester thereof is reduced to the correspondingly substituted 3-hydroxymethyldibenzo[b,f]thiepin by treatment with an alkali metal aluminum hydride, preferably lithium aluminum hydride or borane. Either reagent is effective in reducing a carboxy or a carbalkoxy substituent to the corresponding hydroxymethyl substituent but borane is preferred for the reduction of the carboxyl function in a dibenzothiepin-3-carboxylic acid, 5,5-dioxide. The reduction is conducted in a solvent which is inert under the reaction conditions and may be either a volatile or non-volative ether. Ethyl ether or other loweralkyl ethers are satisfactory and tetrahydrofuran is a commonly preferred solvent.

The reducing reagent, either lithium aluminum hydride or borane, is employed in a slight excess over the stoichiometric amount needed in order to insure completeness of reaction. The reduction reaction is maintained at a temperature between 0° and 100°C. or the reflux temperature of the solvent. Most acids and esters are reduced relatively completely at room temperature (25°C.) and in a relatively short time (approximately 1 hour), and therefore the reduction is usually conducted by stirring the acid or ester for a period of from 30 minutes to 12 hours at a temperature of from 10° to 40°C. Following the completion of the reaction, the product is isolated by the addition of water and extraction into ether. The ether extract is evaporated to obtain the residual product which is recrystallized from an appropriate solvent.

Products which may be obtained directly by reduction of the appropriate acid are 3-hydroxy-

methyldibenzo-[b,f]thiepin and 3-hydroxymethyldibenzo[b,f]thiepin 5,5-dioxide or derivatives bearing $R_1$ and $R_2$ substituents which are resistant to the reducing effect of lithium aluminum hydride or borane. Ordinarily the 3-hydroxymethyldibenzo[b,f]thiepin 5-oxide or $R_1$,$R_2$ derivatives thereof are prepared by oxidation of the corresponding 3-hydroxymethyldibenzo[b,f]thiepin with organic peroxides such as peroxy acids like m-chloroperbenzoic acid. The oxidation can be carried further, if an additional equivalent of acid is employed, to produce the corresponding dibenzo[b,f]thiepin-5,5-dioxides. It will be apparent to one skilled in the art that variations in these preparative schemes will allow one to prepare a variety of substituted 3-hydroxymethyldibenzo[b,f]thiepines, as well as the corresponding thiepin-5-oxides and the thiepin-5,5-dioxides.

For example, 8-amino-3-hydroxymethyldibenzo[b,f]-thiepin is prepared by redution of the correspondingly substituted acid or by catalytic hydrogenation of 3-hydroxymethyl-8-nitro-dibenzo[b,f]thiepin.

The dibenzo[b,f]thiepins of formula Ia of this invention are prepared according to the following general reaction scheme:

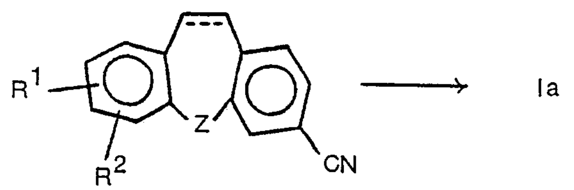

wherein $R^1$, $R^2$, and Z are as defined above.

As shown in the above reaction scheme, an appropriately substituted 3-cyano-dibenzo[b,f]thiepin is reduced and hydrolyzed to the correspondingly substituted dibenzo[b,f]thiepin-3-carboxaldehyde in the presence of a metal containing reducing agent in accordance with the following procedures.

In one method of effecting the conversion of the 3-cyano substituent to a 3-carboxaldehyde substituent, the 3-cyano-dibenzo[b,f]thiepin is treated in dry ether with hydrogen chloride gas and anhydrous stannous chloride at a temperature of 0—50°C., but preferably between 0—10°C. Solvents for the reaction are ethers or other inert anhydrous solvents which do not contain an active hydrogen and which will dissolve the reactants. The intermediate which first forms is the imino chloride salt of the amine which is reduced by the action of stannous chloride to the intermediate tin halide complex. This intermediate aldiminium stannic chlorite complex is then hydrolyzed to produce the desired dibenzo[b,f]thiepin-3-carboxaldehyde. In order to isolate the aldehyde from the tin complex, it is sometimes necessary to utilize a chromatographic procedure and/or a second hydrolytic step using an aqueous alcohol solution.

In an alternate procedure for converting 3-cyanodibenzo[b,f]thiepins to dibenzo[b,f]thiepin-3-carboxaldehydes and the corresponding 10,11-dihydro compounds, the selected cyano compound is treated with from 1—5 parts by weight of Raney alloy (50% Ni/50% Al) in aqueous formic acid. The mixture is stirred at temperatures from 0°C. to reflux temperature, but preferably at the reflux temperature for a period of from 1—24 hours. When the reduction and hydrolysis is complete, the product is recovered by removal of the alloy by filtration while hot, and extraction of the product from the filtrate, followed by crystallization from ethyl acetate.

In a further alternate procedure for the preparation of dibenzo[b,f]thiepin-3-carboxaldehydes or the corresponding 10,11-dihydro derivative, the selected 3-cyanodibenzo[b,f]thiepin or the 10,11-dihydro derivative employed as starting material is reduced to the corresponding aldehyde by selective reduction with sodium hypophosphite and Raney nickel in aqueous acid (preferably aqueous acetic acid or mixtures containing pyridine in aqueous acetic acid). The reaction is preferably conducted at 25°C., although the temperature may be maintained at from 0—100°C. to moderate the speed of reaction and/or to increase the yield of product obtained. The product is readily recovered from the reaction mixture after removal of the Raney nickel catalyst by filtration, followed by washing with ethyl acetate to extract any occluded product. The combined filtrate and washings containing the product are then extracted with a mixture of ethyl acetate and ether and the product obtained as a solid residue by evaporation of the solvents from product-containing extract.

In a further alternate procedure for preparing dibenzo[b,f]thiepin-3-carboxaldehydes, the corresponding 10,11-dihydro-3-hydroxymethyldibenzo[b,f]thiepin, or a 10,11-dihydro compound is oxidized by means of pyridinium chlorochromate in an inert solvent such as dichloromethane, at a temperature between 0°C. and reflux temperature, but preferably at room temperature, for a period of 1—24 hours. When oxidation is complete, the mixture is filtered through a bed of Florisil® and then washed through with the solvent. Evaporation of the filtrate affords the dibenzo[b,f]thiepin-3-carboxyaldehyde which may be purified by chromatography and/or recrystallization as may be appropriate.

A further alternate procedure is employed when the starting material contains a reducible substituent such as a nitro group. In such instances, the substituted e.g., 8-nitro-dibenzo[b,f]thiepin-3-carboxylic acid chloride is reduced with lithium tri-tert-butoxy-aluminum hydride in tetrahydrofuran at −78°C.

Products which may be obtained directly by reduction of the appropriate nitrile are dibenzo[b,f]rthiepin-3-carboxaldehyde and dibenzo[b,f]thiepin-3-carboxaldehyde 5,5-dioxide or derivatives bearing $R_1$ and $R_2$ substituents which are resistant to the reducing effect of the reaction conditions employed. Ordinarily the dibenzo[b,f]thiepin 5-oxide or $R_1$,$R_2$ derivatives thereof are prepared by oxidation of the corresponding dibenzo[b,f]thiepin with organic peroxides such as peroxy acids like m-chloroperbenzoic acid. The oxidation can be carried further, if an additional equivalent of acid is employed, to produce the corresponding dibenzo[b,f]thiepin-5,5-dioxides. It will be apparent to one skilled in the art that variations in these preparative schemes will allow one to prepare a variety of substituted dibenzo[b,f]thiepin-3-carboxaldehydes, as well as the corresponding thiepin-5-oxides and the thiepin-5,5-dioxides.

Preparation of Starting Materials

As described in greater detail in United States Application Serial No. 917,212 filed June 23, 1978, an appropriately substituted mercaptobenzoic acid II is reacted with m-dibromobenzene III ($R_3$ = Br) to obtain the 0-(3-bromophenylthio)benzoic acid IV. Or alternatively, an appropriately substituted o-bromobenzoic acid II ($R_2$ = Br) is reacted with m-bromobenzethiol III ($R_3$ = SH) to give IV:

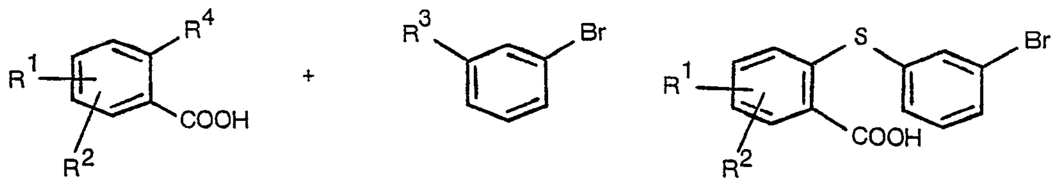

II III IV

where $R_1$ or $R_2$ are each selected from hydrogen, nitro, amino, $C_1$ to $C_4$ alkanoyl, hydroxyl, $C_1$ to $C_4$ alkoxy, $C_1$ to $C_4$ alkylsulfinyl, $C_1$ to $C_4$ alkylsulfonyl, and $C_1$ to $C_4$ alkyl, trifluoromethyl, and trifluoromethylthio. $R_2$ and $R_3$ are different and alternatively are thiol and bromo.

Generally, the sulfide-forming reaction is carried out according to the methods described by Jilek et al., MONATSH. CHEM. *96*, 200 (1965); Protiva et al., Czechoslovakian Patent 121,337, CHEM. ABSTRACTS *68*: 105, 247t (1968); and U.S. Patent 3,711,489; and by other procedures well known in the art.

The resulting o-(3-bromophenylthio)benzoic acid (IV) is reduced to the alcohol, brominated, and the bromo replaced with cyano. The cyano derivative is then hydrolyzed to the carboxylic acid V.

IV

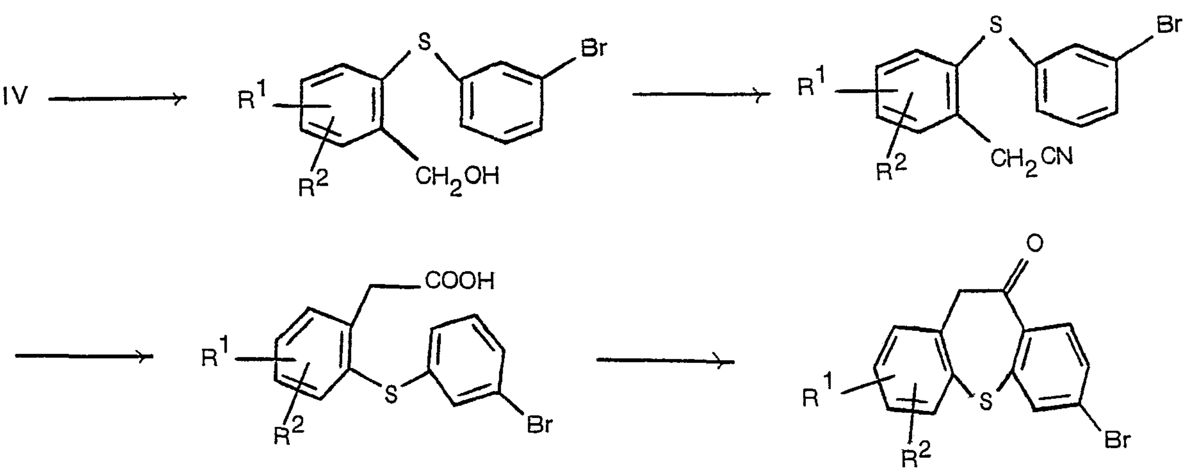

V VI

The carboxylic acid V is transformed into the 3-bromo-11-oxo-10,11-dihydrodibenzo[b,f]thiepin by first conversion to the acid halide with thionyl or phosphoryl halide followed by Friedel-Crafts cyclization with a Lewis acid such as aluminum chloride to give VI. Reduction of the ketone VI with alkali metal borohydrides to the alcohol VII, followed by heating in an appropriate solvent such as toluene with catalytic amounts of a mineral acid, such as sulfuric acid or toluenesulfonic acid provides the 3-bromodibenzo[b,f]thiepin VIII.

VI 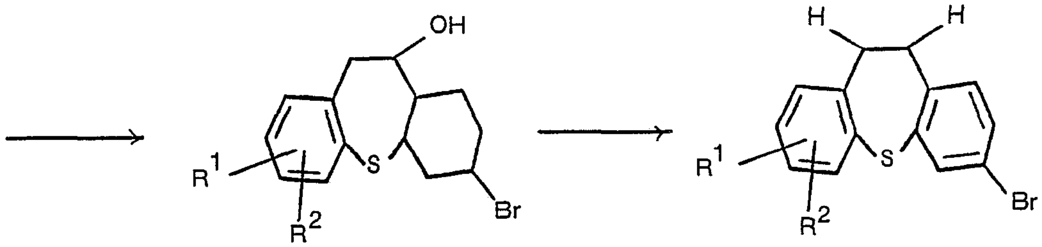

VII VIII

The 3-bromo derivative VIII is then converted to the 3-nitrile IX by reaction with cuprous cyanide in a high boiling polar solvent such as dimethylformamide, N-methylpyrrolidone.

VIII 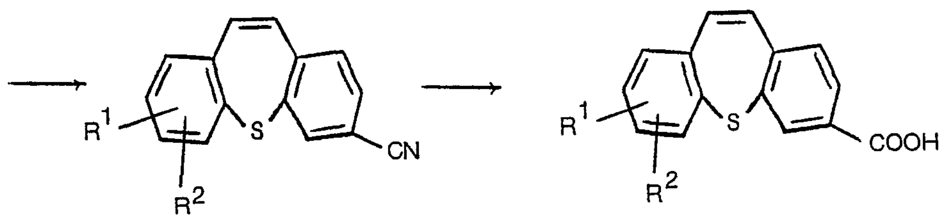

IX X

The 3-cyano derivative IX may be hydrolyzed with aqueous mineral acid or base to give the dibenzo[b,f]thiepin-3-carboxylic acid X. Alternatively, the cyano intermediate IX may be oxidized with organic peroxides such as peroxy acids, for example, m-chloroperbenzoic acid in a stepwise fashion to the corresponding sulfoxide XI and sulfone XII, controlling the molar ratio of oxidant to reductant. This determines the oxidation level of the sulfur. For example, a 1:1 molar ratio results largely in the production of sulfoxide XI. In contrast, a 2:3 molar excess of oxidant results in a yield predominantly comprising the sulfone XII.

VI 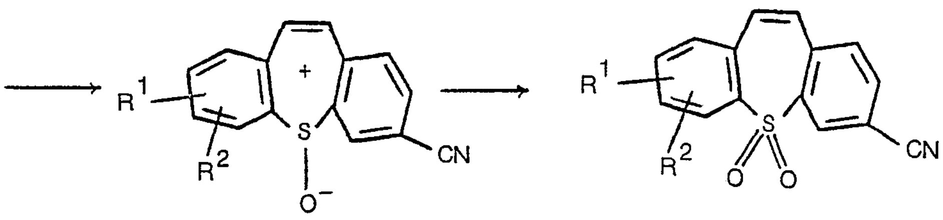

XI XII

Hydrolysis of XI and XII using aqueous mineral acid or alkali provides the corresponding carboxylic acid XIII and XIV.

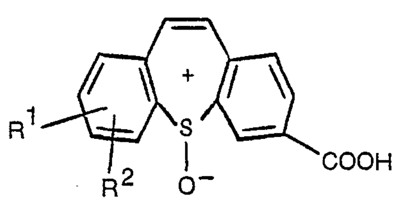

XIII

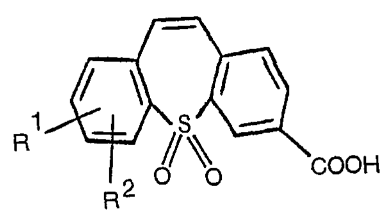

XIV

Compounds of type I where the 10,11-double bond is saturated are prepared from intermediates VII, by displacement of the hydroxy using $PBr_3$, and reduction of the resulting 3,11-dibromide XV by $NaBH_4$ in a solvent such as sulfolane to yield the 3-bromo-10,11-dihydro derivative XVI.

VII

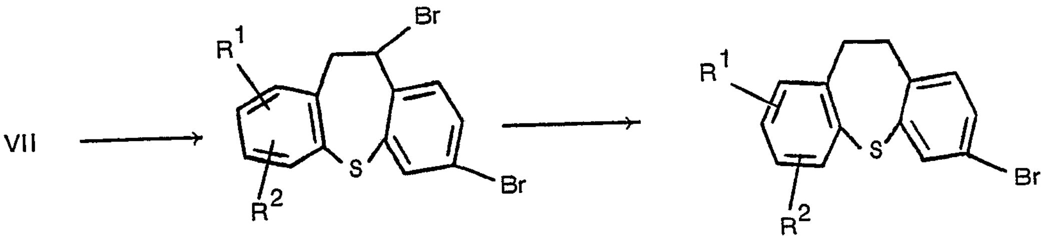

XV XVI

The bromine in XVI can be displaced by cuprous cyanide in a high-boiling polar solvent such as dimethylformamide, and the resulting 3-nitrile XVII hydrolyzed with aqueous mineral acid or base to give the 10,11-dihydrodibenzo[b,f]thiepin-3-carboxylic acid XVIII.

XVI

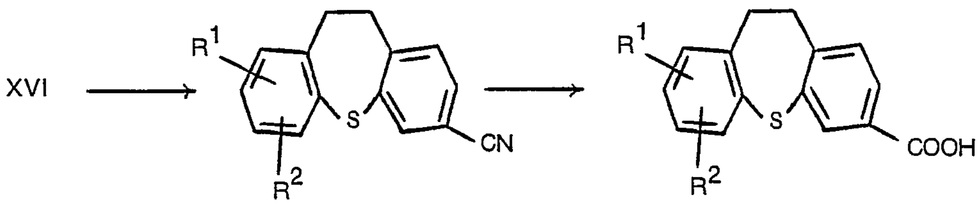

XVII XVIII

Compound XVII can be oxidized with one equivalent of an organic peroxide, such as m-chloroperbenzoic acid, to yield the sulfoxide XIX which can then be hydrolyzed with mineral acid or base to the carboxylic acid XX. Compound XVII may also be oxidized with two equivalents of the oxidizing agent to afford the corresponding sulfone XXI, which on hydrolysis gives the acid XXII.

XVII

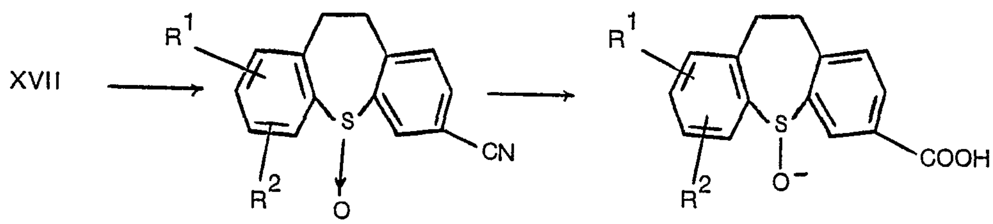

XIX XX

XVII

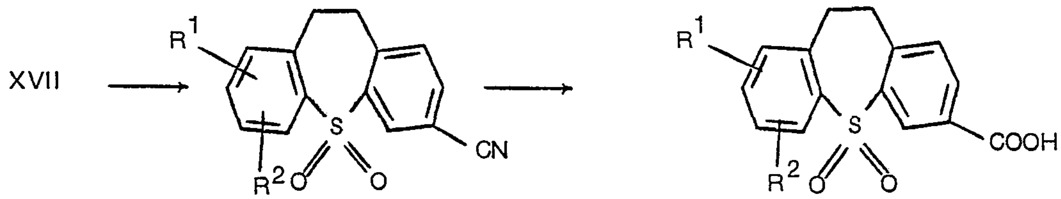

XXI XXII

Substituent $R_1$ in I ($R_2 = H$) can also be introduced by modification of the nitro group in IX or XVII ($R_1 = NO_2$, $R_2 = H$) by known procedures. For example, XXIII can be reduced with stannous chloride in acidic medium, hydrochloric acid to yield XXIV which can be hydrolyzed with mineral acids or bases to XXV.

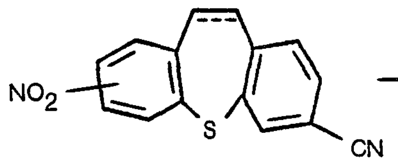

XXIII

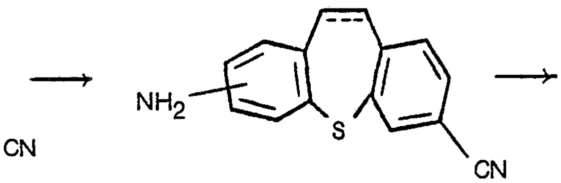

XXIV

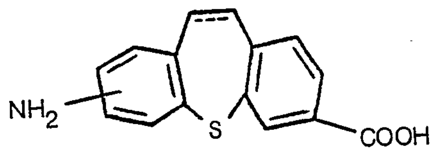

XXV

Alternatively, XXIII may be oxidized with peroxides, for example, m-chloroperbenzoic acid to yield XXVI which can be reduced to XXVII and then hydrolyzed with mineral acids or bases to XXVIII.

XXIII

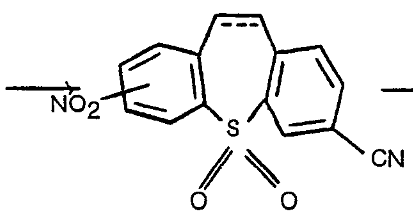

XXVI

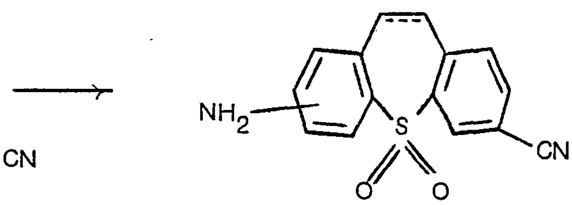

XXVII

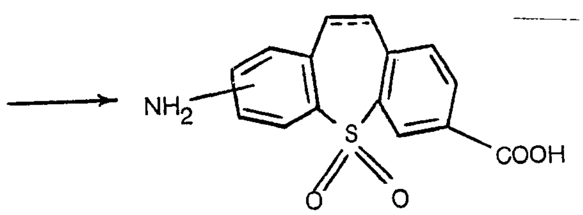

XXVIII

Intermediate XXIV can be reacted with sodium nitrite in mineral acid to the diazonium salt XXIX, where X is a mineral acid counter ion, for example, $Cl^-$, $HSO_4^-$, $BF_4^-$, which on reaction with CuCl and $CuCl_2$ yields intermediate XXX which can be hydrolyzed to the acid XXXI. Intermediate XXX may also be oxidized to the sulfone derivative XXXII, then followed by a hydrolysis to the carboxylic acid XXXIII.

XXIV 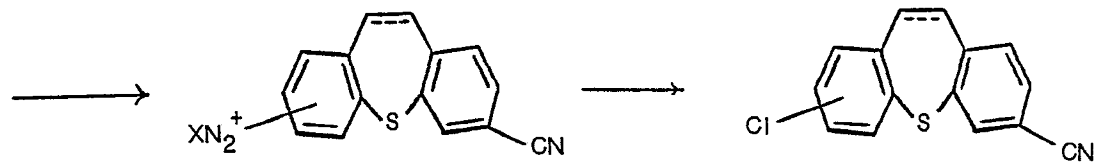

XXIX XXX

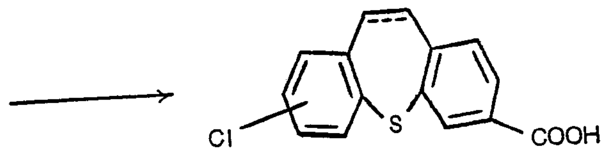

XXXI

XXX 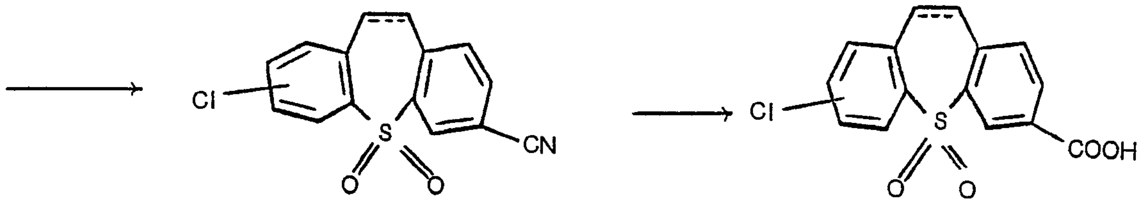

XXXII XXXIII

Derivative XXIX can be hydrolyzed with a solution of sulfuric acid 10 to 50% in strength at temperatures ranging from 0° to 90°C. to yield XXXIV. XXIX may also be reacted with potassium thioxanthate at temperatures from 40° to 70°C. followed by basic hydrolysis to yield the thiol acid XXXV.

XXIX 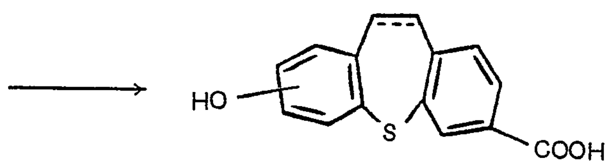

XXXIV

XXIX 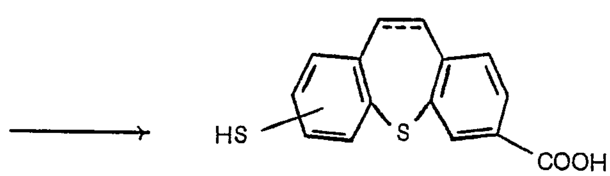

XXXV

Compound XXVII can be transformed in the usual manner to the diazonium salt XXXVI, which can be reacted as described above to yield compounds XXXVII and XXXVIII.

XXVII 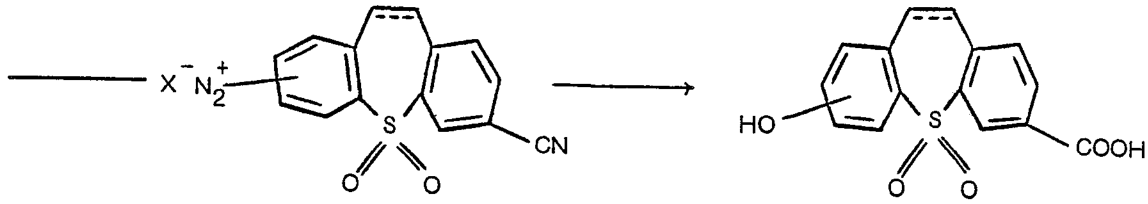

XXXVI XXXVII

XXXVI 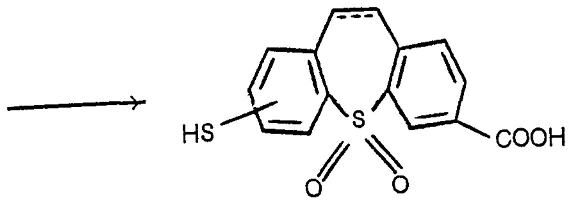

XXXVIII

Compounds XXXIV, XXXV, XXXVII, and XXXVIII can be reacted with alkyl halides RS in which R is a loweralkyl $C_1$ to $C_4$, benzyl, and X is a leaving group such as Cl, Br, I, and

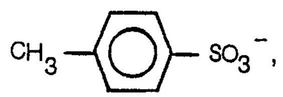

in the presence of bases such as alkali carbonate, hydroxides in solvents such as dimethylformamide, at temperatures ranging from 30° to 160°C. to yield XXXIX, XL, XLI, and XLII, respectively.

XXXIV 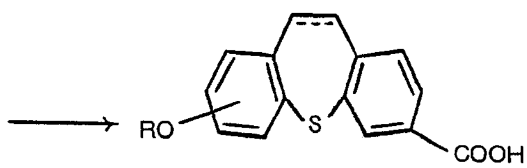

XXXIX

XXXIV 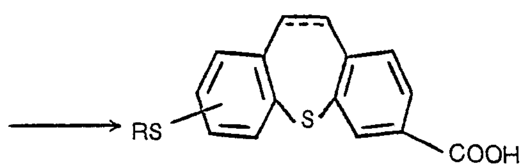

XL

XXXVII 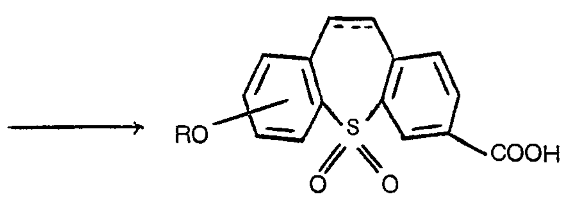

XLI

XXXVIII

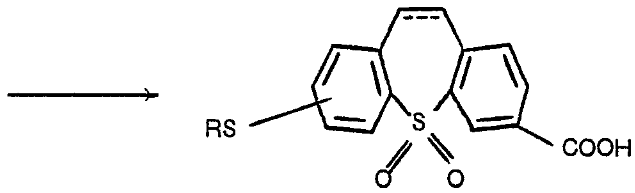

XLII

Compound XL can in a controlled oxidation with peroxides such as hydrogen peroxide or organic peroxy acids such as m-chloroperbenzoic acid, yield compound XLIII. XLII may be oxidized with one equivalent of organic peroxides such as m-chloroperbenzoic acid or with hydrogen peroxide in hydroxylic solvents such as alcohols, organic acids such as acetic acid, at temperatures below 30°C., to yield XLIV. Compounds XL, XLII, and XLIV may also be oxidized with excess organic peroxides such as m-chloroperbenzoic acid at room temperature, or with peroxides such as hydrogen peroxide in acidic medium such as acetic acid at temperatures between 80° and 100°C. to yield XLV.

XL

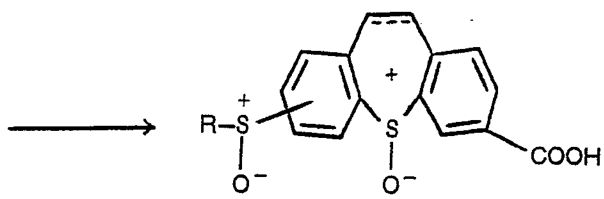

XLIII

XLVI

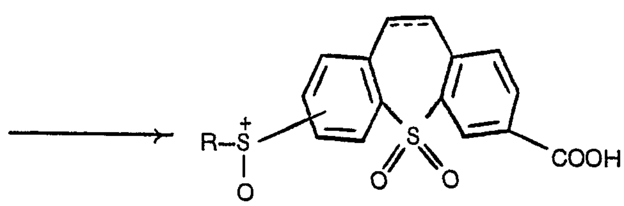

XLIV

XL, XLII, XLIV

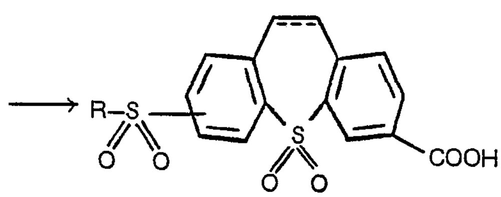

XLV

Specific introduction of substituents in position 8 in I can also be achieved as follows. For example, XVII ($R_1 = R_2 = H$) can react with alkanoyl halide RCOX or alkanoic anhydride RCOOCOR in which R is a loweralkyl $C_1$ to $C_4$ and X is chloro or bromo, under Friedel-Crafts conditions, to yield the 8-substituted acyl derivative XLVI; the 10, 11-bridge may be brominated with a brominating agent such as N-bromosuccinimide, and treatment with a base such as DBN can generate the 10,11-unsaturated derivative XLVII.

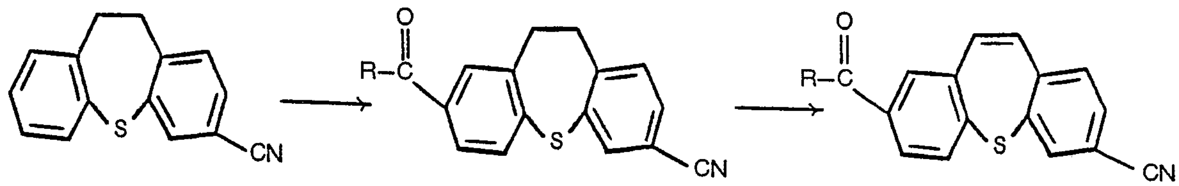

XVII XLVI XLVII

Both XLVI and XLVII can be hydrolyzed with mineral acid or base to the corresponding acids XLVIII and XLIX.

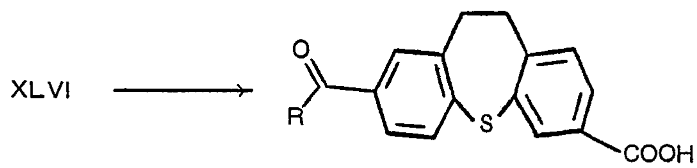

XLVI

XLVIII

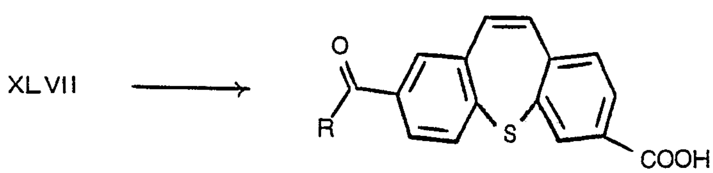

XLVII

XLIX

XLVII can be oxidized with oxidizing agents such as m-chloro perbenzoic acid stepwise to yield sulfoxide L and sulfone LI which are hydrolyzed under acidic or basic conditions to afford acids LII and LIII respectively.

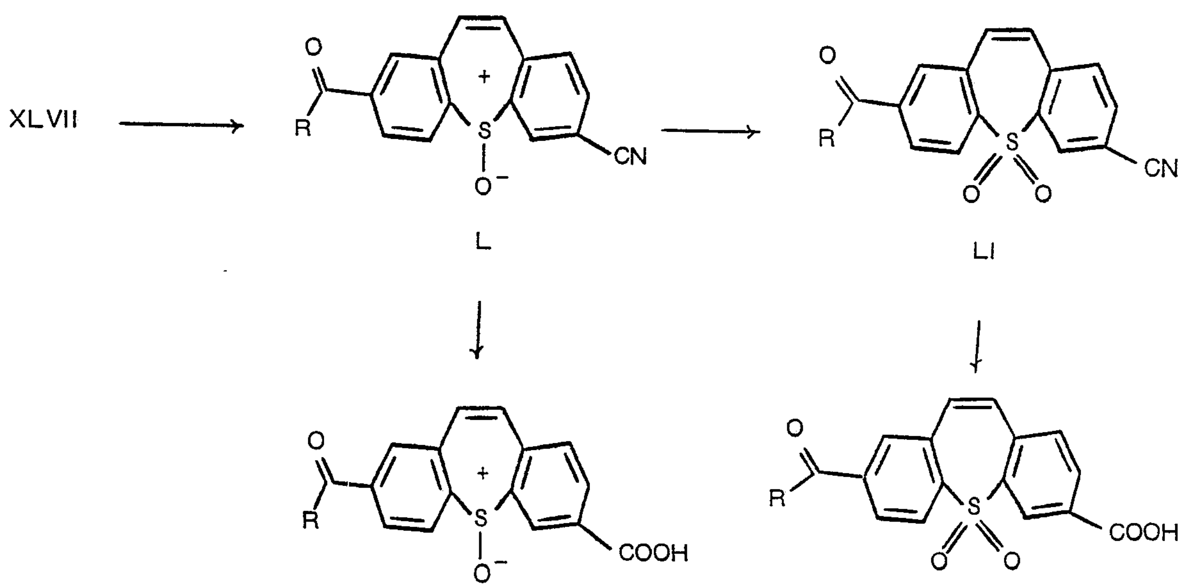

XLVII

L LI

LII LIII

Compound XLIX can be reacted with hydroxylamine hydrochloride with presence of base to yield oxime LIV which on a Beckman rearrangement, yields the acylamino compound LV which upon hydrolysis yields amino acid LVI.

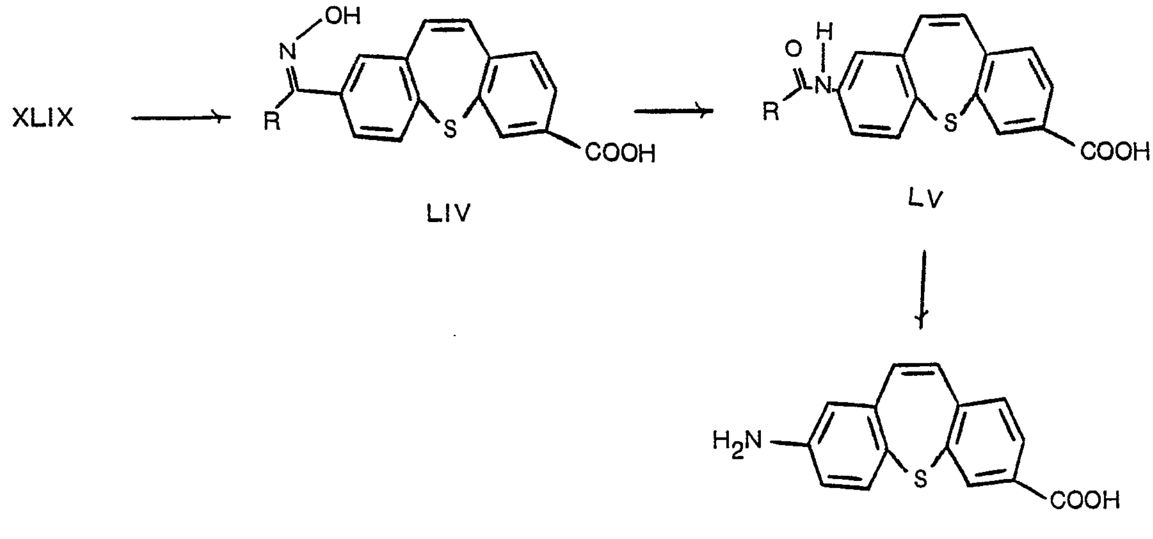

LIV

LV

LVI

Compound LV can be oxidized with hydrogen peroxide in acetic acid stepwise to yield the corresponding sulfoxide LVII and sulfone LVIII which upon hydrolysis afford the acids LIX and LX.

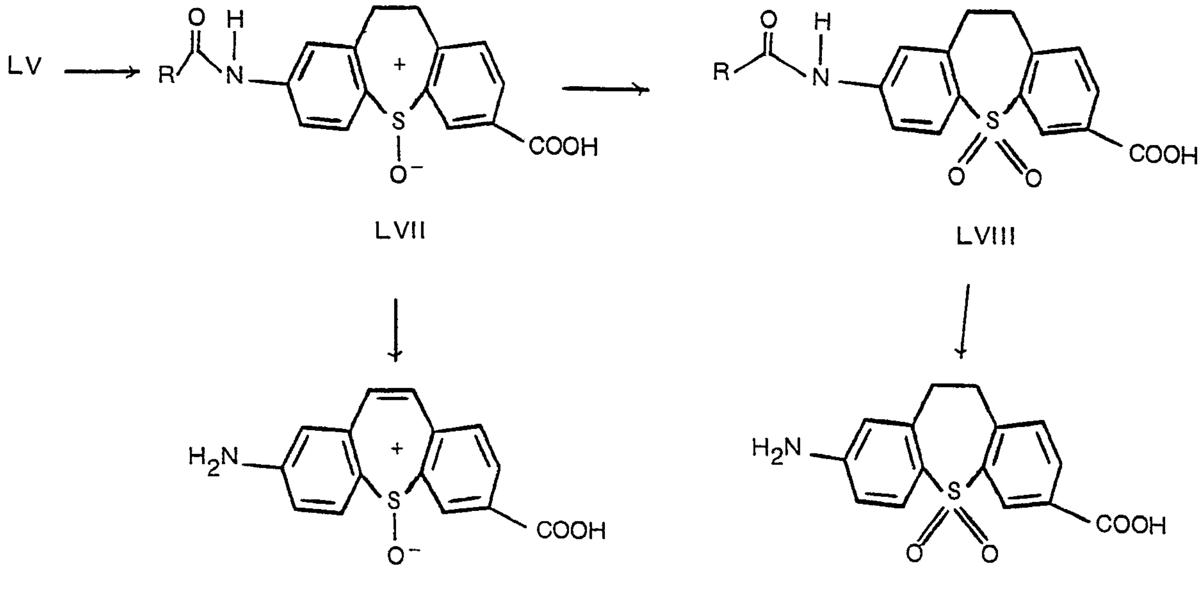

LVII

LVIII

LIX

LX

Compounds LVI, LIX, and LX can be treated in various Sandmeyer reactions as described earlier to yield I substituted in the 8 position.

Compound XLIX ($R = CH_3$), when treated with sodium hypochlorite and base at temperatures from 0° to 70°C. for half an hour, yield the diacid XLI. When the process is carried for two days under the same conditions, XLII is obtained.

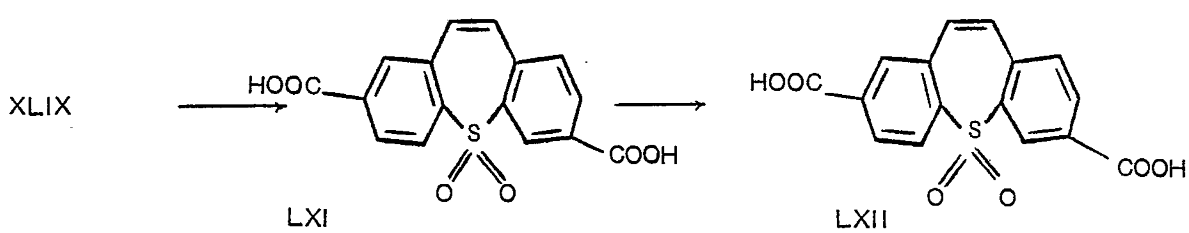

LXI

LXII

Compounds XLIX, LII, and LXIII can be reduced with sodium borohydride to afford the corresponding alcohols, XLIII, LXIV, and LXV.

XLIX

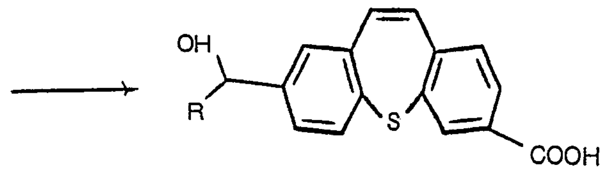

LXIII

LII

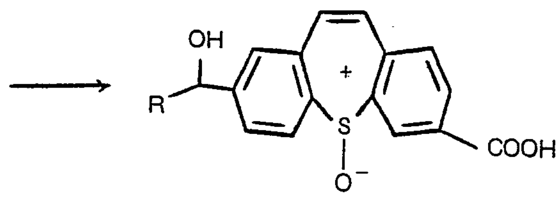

LXIV

LIII

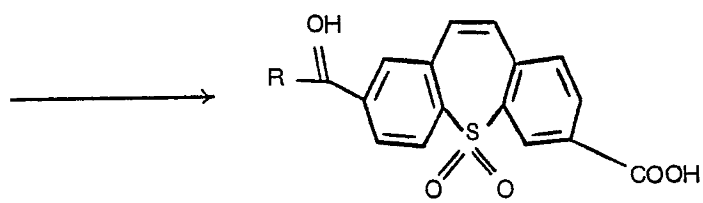

LXV

The higher homologs of Compound X hereinabove are prepared, starting with the appropriately substituted 3-carboxylic acid, through reduction, bromination, cyanization, and oxidation, to afford the corresponding 3-acetic acid derivative. Quite obviously, the described reduction, bromination, cyanization, and oxidation sequence can be repeated, employing the 3-acetic acid derivative as starting material, in order to obtain the corresponding propionic acid derivative which, in turn, can be employed as starting material for preparing the corresponding butyric acid derivative. In this manner, any desired 3-loweralkanoic acid derivative is readily prepared. Corresponding sulfinyl or sulfonyl derivatives are prepared by the oxidation techniques previously described.

Compounds of type IA where the 10,11 double bond is saturated are prepared from intermediate V in which the 3-bromo is converted to a cyano derivative XVIIA.

V

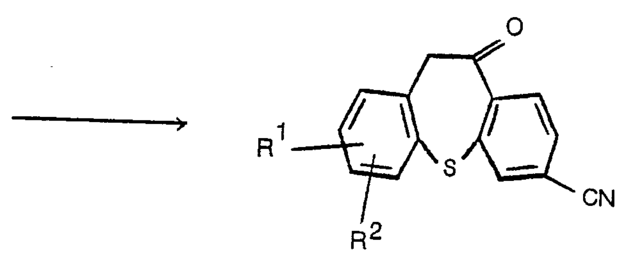

XVIIA

The compound XVIIA is reduced by conventional methods, e.g., Wolff-Kishner, to compound XXII.

XVIIA 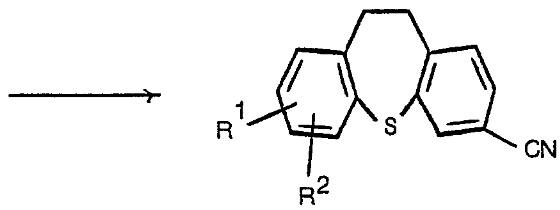

XXIIA

Substituent R in IA can also be introduced by modification of the nitro group in IX ($R = NO_2$) by known procedures. For example, XXXIIIA can be reduced with stannous chloride in acidic medium, hydrochloric acid, and the like, to yield XXXIVA which can be hydrolyzed with mineral acids or bases to XXXV.

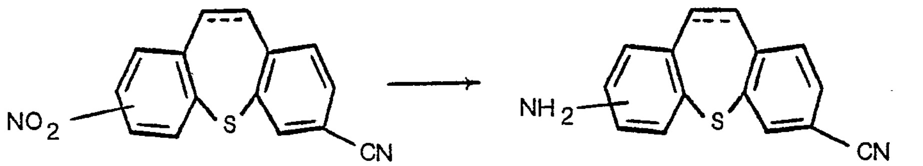

XXXIIIA XXXIVA

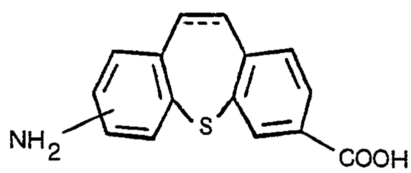

XXXVA

Alternatively, XXXIIIA may be oxidized with peroxides, for example, m-chloroperbenzoic acid to yield XXXVIA which can be reduced to XXXVIIA and then hydrolyzed with mineral acids or bases to XXXVIIIA.

XXXIIIA 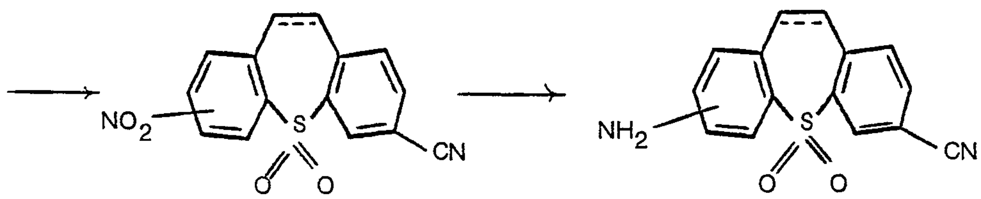

XXXVIA XXXVIIA

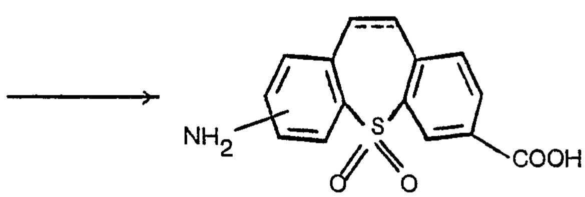

XXXVIIIA

Intermediate XXXIVA can be reacted with sodium nitrite in mineral acid to the diazonium salt XXXIXA, where X is a mineral acid counter ion, for example, $Cl^-$, $HSO_4^-$, $BF_4^-$, which on reaction with CuCl and $CuCl_2$ yields intermediate XLA which can be hydrolyzed to the acid XLIA. Intermediate XLA may also be oxidized to the sulfone derivative.

XXXIVA 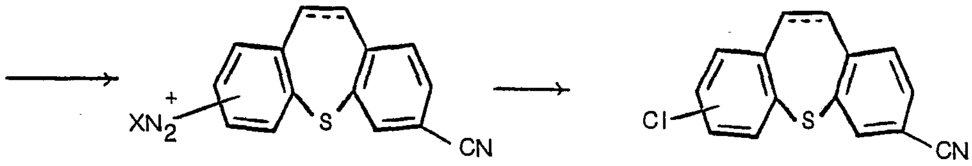

XXXIXA XLA

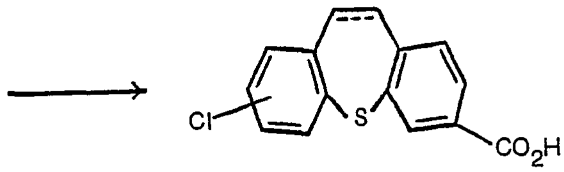

XLIA

Compound XXXVIIA can be transformed in the usual manner to the diazonium salt XLVA.

XXXVIIA 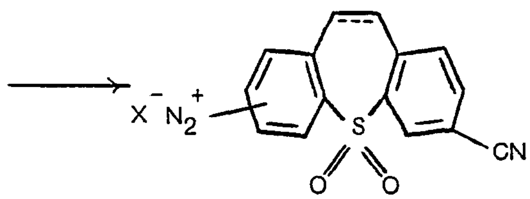

XLVA

The 3-hydroxymethyldibenzo[b,f]thiepin and the 10,11-dihydro compounds which may be used as intermediates for the preparation of the aldehyde compounds may be made by reduction of the corresponding dibenzo[b,f]thiepin-3-carboxylic acids or esters as described in detail in United States Application Serial No. 917,212, filed June 23, 1978.

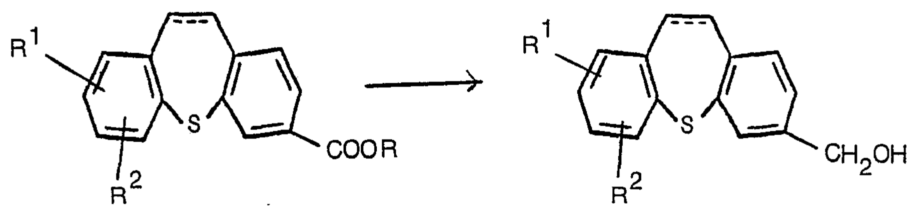

These alcohols may be oxidized to the sulfinyl or sulfonyl oxidation levels by a peroxy acid, for example m-chloroperbenzoic acid in a stepwise fashion.

Example 1

*3-Hydroxymethyl-10,11-dihydrodibenzo[b,f]thiepin*

Dissolve 7.5 gm. of 10,11-dihydrodibenzo[b,f]thiepin-3-carboxylic acid in 140 ml. of tetrahydrofuran. With stirring, add 90 ml. of 1N diborane in tetrahydrofuran. Stir at room temperature for 2 hours and dilute the reaction mixture with water. Extract into ether, and evaporate the ether extract to dryness. Stir the residue with hexane and filter to obtain the title product (m.p., 82—84°C.).

Example 2

*3-Hydroxymethyl-10,11-dihydrodibenzo[b,f]thiepin-5-oxide*

Dissolve 2 gm. of 3-hydroxymethyl-10,11-dihydrodibenzo[b,f]thiepin in 200 ml. of methylene chloride at room temperature. Add 1.7 gm. of m-chloroperbenzoic acid. Stir at room temperature for 2 hours. Add 4 gm. of calcium hydroxide and 50 ml. of methylene chloride. Continue stirring for 15 minutes. Filter through celite evaporate the filtrate to dryness. Crystallize the residue from toluene to obtain the title product (m.p., 144—146°C.).

Example 3

*3-Hydroxymethyl-10,11-dihydrodibenzo[b,f]thiepin-5,5-dioxide*

Repeat the process of Example 6, substituting an equivalent quantity of 10,11-dihydrodibenzo[b,f]thiepin-3-carboxylic acid-5,5-dioxide for the dibenzo[b,f]thiepin-3-carboxylic acid-5,5-dioxide, to obtain the title product (m.p., 128—130°C.).

Example 4

*3-Hydroxymethyldibenzo[b,f]thiepin*

Repeat the process of Example 1, substituting an equivalent quantity of dibenzo[b,f]thiepin-3-carboxylic acid for the 10,11-dihydrodibenzo[b,f]thiepin-3-carboxylic acid, to obtain the title product (m.p., 111—112°C.).

Example 5

*3-Hydroxymethyldibenzo[b,f]thiepin-5-oxide*

Repeat the process of Example 2, substituting an equivalent quantity of 3-hydroxymethyl-dibenzo[b,f]-thiepin for the 3-hydroxymethyl-10,11-dihydrodibenzo[b,f] thiepin, to obtain the title product (m.p., 146—148°C.).

Example 6

*3-Hydroxymethyldibenzo[b,f]thiepin-5,5-dioxide*

Mix 2.8 gm. of dibenzo[b,f]thiepin-3-carboxylic acid-5,5-dioxide, 50 ml. of tetrahydrofuran, and 30 ml. of 1N diborane in tetrahydrofuran. Stir at room temperature for 1 hour. Dilute the reaction mixture with water, and extract into ether. Evaporate the ether extract to dryness. Stir the residue in ether, and evaporate to dryness to obtain the title product (m.p., 156—158°C.).

Example 7

*3-Hydroxymethyl-8-nitro, dibenzo[b,f]thiepin*

Repeat the process of Example 1, substituting an equivalent quantity of 8-nitro-dibenzo[b,f]thiepin-3-carboxylic acid for the 10,11-dihydrodibenzo[b,f]thiepin-3-carboxylic acid, to obtain the title product.

Example 8

*3-Hydroxymethyl-8-nitro-dibenzo[b,f]thiepin-5-oxide*

Repeat the process of Example 2, substituting an equivalent quantity of 3-hydroxymethyl-8-nitro-dibenzo[b,f]thiepin for the 3-hydroxymethyl-10,11-dihydrodibenzo[b,f]thiepin, to obtain the title product.

Example 9

*3-Hydroxymethyl-8-nitro-dibenzo[b,f]thiepin 5,5-dioxide*

Repeat the process of Example 6, substituting an equivalent quantity of 8-nitro-dibenzo[b,f]thiepin-3-carboxylic acid 5,5-dioxide for the dibenzo[b,f]thiepin-3-carboxylic acid 5,5-dioxide, to obtain the title product.

Example 10

*8-Amino-3-hydroxymethyl-dibenzo[b,f]thiepin*

Repeat the process of Example 1, substituting an equivalent quantity of 8-amino-dibenzo[b,f]thiepin-3-carboxylic acid for the 10,11-dihydrodibenzo[b,f]thiepin-3-carboxylic acid, to obtain the title product.

Example 11

*8-Amino-3-hydroxymethyl-dibenzo[b,f]thiepin 5,5-dioxide*

Repeat the process of Example 6, substituting an equivalent quantity of 8-amino-dibenzo[b,f]thiepin-3-carboxylic acid 5,5-dioxide for the dibenzo[b,f]thiepin-3-carboxylic acid 5,5-dioxide, to obtain the title product.

Example 12

*8-Chloro-3-hydroxymethyl-dibenzo[b,f]thiepin*

Repeat the process of Example 1, substituting an equivalent quantity of 8-chloro-dibenzo[b,f]thiepin-3-carboxylic acid for the 10,11-dihydro-dibenzo[b,f]thiepin-3-carboxylic acid, to obtain the title product.

Example 13

*8-Chloro-3-hydroxymethyl-dibenzo[b,f]thiepin 5,5-dioxide*

Repeat the process of Example 6, substituting an equivalent quantity of 8-chloro-

dibenzo[b,f]thiepin-3-carboxylic acid 5,5-dioxide for the dibenzo[b,f]thiepin-3-carboxylic acid 5,5-dioxide, to obtain the title product.

Example 14

*8-Hydroxy-3-hydroxymethyl-dibenzo[b,f]thiepin*

Repeat the process of Example 1, substituting an equivalent quantity of 8-hydroxy-dibenzo[b,f]thiepin-3-carboxylic acid for the 10,11-dihydro-dibenzo[b,f]thiepin-3-carboxylic acid, to obtain the title product.

Example 15

*8-Hydroxy-3-hydroxymethyl-dibenzo[b,f]thiepin 5,5-dioxide*

Repeat the process of Example 6, substituting an equivalent quantity of 8-hydroxy-dibenzo[b,f]thiepin-3-carboxylic acid 5,5-dioxide for the dibenzo[b,f]thiepin-3-carboxylic acid 5,5-dioxide, to obtain the title product.

Example 16

*3-Hydroxymethyl-8-mercapto-dibenzo[b,f]thiepin*

Repeat the process of Example 1, substituting an equivalent quantity of 8-mercapto-dibenzo[b,f]thiepin-3-carboxylic acid for the 10,11-dihydro-dibenzo[b,f]thiepin-3-carboxylic acid, to obtain the title product.

Example 17

*3-Hydroxymethyl-8-mercapto-10,11-dihydrodibenzo[b,f]thiepin 5,5-dioxide*

Repeat the process of Example 6, substituting an equivalent quantity of 8-mercapto-10,11-dihydrodibenzo[b,f]thiepin-3-carboxylic acid 5,5-dioxide for the dibenzo[b,f]thiepin-3-carboxylic acid 5,5-dioxide, to obtain the title product.

Example 18

*3-Hydroxymethyl-8-methoxy-dibenzo[b,f]thiepin*

Repeat the process of Example 1, substituting an equivalent quantity of 8-methoxy-dibenzo[b,f]thiepin-3-carboxylic acid for the 10,11-dihydrodibenzo[b,f]thiepin-3-carboxylic acid, to obtain the title product.

Example 19

*3-Hydroxymethyl-8-methoxy-dibenzo[b,f]thiepin 5,5-dioxide*

Repeat the process of Example 6, substituting an equivalent quantity of 8-methoxy-dibenzo[b,f]thiepin-3-carboxylic acid 5,5-dioxide for the dibenzo[b,f]thiepin-3-carboxylic acid 5,5-dioxide, to obtain the title product.

Example 20

*3-Hydroxymethyl-8-methylthio-dibenzo[b,f]thiepin*

Repeat the process of Example 1, substituting an equivalent quantity of 8-methylthiodibenzo[b,f]thiepin-3-carboxylic acid for the 10,11-dihydrodibenzo[b,f]thiepin-3-carboxylic acid, to obtain the title product.

Example 21

*3-Hydroxymethyl-8-methylthio-dibenzo[b,f]thiepin 5,5-dioxide*

Repeat the process of Example 6, substituting an equivalent quantity of 8-methylthiodibenzo[b,f]thiepin-3-carboxylic acid 5,5-dioxide for the dibenzo[b,f]thiepin-3-carboxylic acid 5,5-dioxide, to obtain the title product.

Example 22

*3-Hydroxymethyl-8-methylsulfinyl-dibenzo[b,f]thiepin 5,5-dioxide*

Repeat the process of Example 6, substituting an equivalent quantity of 8-methylsulfinyl-dibenzo[b,f]thiepin-3-carboxylic acid 5,5-dioxide for the dibenzo[b,f]thiepin-3-carboxylic acid 5,5-dioxide, to obtain the title product.

Example 23

*3-Hydroxymethyl-8-methylsulfinyl-dibenzo[b,f]thiepin 5-oxide*

Repeat the process of Example 2, substituting an equivalent quantity of 3-hydroxymethyl-8-methyliodibenzo[b,f]thiepin for the 10,11-dihydrodibenzo[b,f]thiepin, to obtain the title product.

Example 24

*3-Hydroxymethyl-8-methylsulfonyl-dibenzo[b,f]thiepin 5,5-dioxide*

Repeat the process of Example 6, substituting an equivalent quantity of 8-methylsulfonyl-

dibenzo[b,f]thiepin-3-carboxylic acid 5,5-dioxide for the dibenzo[b,f]thiepin-3-carboxylic acid 5,5-dioxide, to obtain the title product.

Example 25

*8-Acetyl-3-hydroxymethyl-10,11-dihydrodibenzo[b,f]thiepin*

Repeat the process of Example 1, substituting an equivalent quantity of 8-acetyl-10,11-dihydrodibenzo[b,f]thiepin-3-carboxylic acid for the 10,11-dihydrodibenzo[b,f]thiepin-3-carboxylic acid, to obtain the title product.

Example 26

*8-Acetyl-3-hydroxymethyl-10,11-dihydrodibenzo[b,f]thiepin 5,5-dioxide*

Repeat the process of Example 6, substituting an equivalent quantity of 8-acetyl-10,11-dihydrodibenzo[b,f]thiepin-3-carboxylic acid 5,5-dioxide for the dibenzo[b,f]thiepin-3-carboxylic acid 5,5-dioxide, to obtain the title product.

Example 27

*3,8-Dihydroxymethyl-10,11-dihydrodibenzo[b,f]thiepin*

Repeat the process of Example 1, substituting an equivalent quantity of 8-hydroxymethyl-10,11-dihydrodibenzo[b,f]thiepin-3-carboxylic acid for the 10,11-dihydrodibenzo[b,f]thiepin-3-carboxylic acid, to obtain the title product.

Example 28

*3,8-Dihydroxymethyl-10,11-dihydrodibenzo[b,f]thiepin 5,5-dioxide*

Repeat the process of Example 6, substituting an equivalent quantity of 8-hydroxymethyl-10,11-dihydrodibenzo[b,f]thiepin-3-carboxylic acid 5,5-dioxide for the dibenzo[b,f]thiepin-3-carboxylic acid 5,5-dioxide, to obtain the title product.

Example 29

*3(2-Hydroxyethyl)-11-oxo-10,11-dihydrodibenzo[b,f]thiepin 5,5-dioxide*

Repeat the process of Example 6, substituting an equivalent quantity of 10,11-dihydro-11-oxo-dibenzo[b,f]thiepin-3-acetic acid 5,5-dioxide for the dibenzo[b,f]thiepin-3-carboxylic acid, to obtain the title product.

Example 30

*8-Fluoro-3-hydroxymethyl-10,11-dihydrodibenzo[b,f]thiepin*

Repeat the process of Example 1, substituting an equivalent quantity of 8-fluoro-10,11-dihydrodibenzo[b,f]thiepin-3-carboxylic acid for the 10,11-dihydrodibenzo[b,f]thiepin-3-carboxylic acid, to obtain the title product (m.p., 80—81°C.).

Example 31

*8-Fluoro-3-hydroxymethyl-10,11-dihydrodibenzo[b,f]thiepin-5-oxide*

Repeat the process of Example 2, substituting an equivalent quantity of 8-fluoro-3-hydroxymethyl-10,11-dihydrodibenzo[b,f]thiepin for the 3-hydroxymethyldibenzo[b,f]thiepin, to obtain the title product (m.p. 181—183°C.).

Example 32

*8-Fluoro-3-hydroxymethyl-10,11-dihydrodibenzo[b,f]thiepin 5,5-dioxide*

Repeat the process of Example 6, substituting an equivalent quantity of 8-fluoro-10,11-dihydrodibenzo[b,f]thiepin-3-carboxylic acid 5,5-dioxide for the dibenzo[b,f]thiepin-3-carboxylic acid 5,5-dioxide, to obtain the title product (m.p., 116—118°C.).

Example 33

*8Fluoro-3-hydroxymethyl-dibenzo[b,f]thiepin*

Repeat the process of Example 1, substituting an equivalent quantity of 8-fluorodibenzo[b,f]thiepin-3-carboxylic acid for the 10,11-dihydrodibenzo[b,f]thiepin-3-carboxylic acid, to obtain the title product (m.p., 123—125°C.).

Example 34

*8-Fluoro-3-hydroxymethyl-dibenzo[b,f]thiepin 5-oxide*

Repeat the process of Example 2, substituting an equivalent quantity of 8-fluoro-3-hydroxymethyldibenzo[b,f]thiepin for the 3-hydroxymethyldibenzo[b,f]thiepin to obtain the title product (m.p., 161—163°C.).

Example 35

*8-Fluoro-3-hydroxymethyl-dibenzo[b,f]thiepin 5,5-dioxide*

Repeat the process of Example 6, substituting an equivalent quantity of 8-fluoro-dibenzo[b,f]thiepin-3-carboxylic acid 5,5-dioxide for the dibenzo[b,f]thiepin-3-carboxylic acid 5,5-dioxide, to obtain the title product (m.p., 131—133°C.).

Example 36

*Dibenzo[b,f]thiepin-3-carboxaldehyde 5,5-dioxide*

Mix 267 mg. (1 mmole) 3-cyano-dibenzo[b,f]thiepin 5,5-dioxide with Raney alloy (500 mg.) and a mixture of 4 ml. formic acid and 1 ml. water at the reflux temperature. After 3 hours, add 200 mg. additional Raney alloy and heat for one hour to obtain complete reduction. Filter the reaction mixture while hot to remove the alloy, and wash with ethyl acetate to remove any occluded solvent. Wash the organic layer containing the product with water and with saturated sodium chloride solution. Separate the organic layer containing the product to produce a residue of a pale yellow oil, which crystallizes in a small volume of ethyl acetate, m.p. 167—171°C.

Example 37

*Dibenzo[b,f]thiepin-3-carboxaldehyde 5,5-dioxide*

Mix 267 mg. (1 mmole) of 3-cyano-dibenzo[b,f]thiepin 5,5-dioxide, 10 ml. pyridine, 5 ml. acetic acid, and 5 ml. water. Add 0.5 g. sodium hypophosphite and approximately 0.1 g. Raney nickel. Stir at 25°C. for 24 hours and then at 45—50°C. for 2 hours. Filter the reaction mixture to remove the catalyst and wash any product from the catalyst using 10 ml. ethyl acetate. Add 10 ml. 6N hydrochloric acid and separate the organic layer containing the product. Evaporate the organic layer to obtain a solid residue comprising the title product, m.p., 175—177°C. Swishing with methanol and drying the residue gives product melting at 177—178°C.

Example 38

*Dibenzo[b,f]thiepin-3-carboxaldehyde*

Saturate a suspension of anhydrous stannous chloride (378 mg; 2 mmole) in 7.5 ml. dry ether with hydrogen chloride at a temperature of 0—5°C., and add 235 mg. (1 mmole) of 3-cyano-dibenzo[b,f]thiepin. Stir for one hour at 0°C. and allow to warm to room temperature (25°C.). Continue stirring at room temperature for approximately 48 hours. Add 5 ml. of water and 1 ml. 2N hydrochloric acid, and extract with a mixture of 5 ml. ether and 5 ml. ethyl acetate. When the hydrolysis of the chloro stannate is complete, evidenced by the disappearance of solids from the solution, separate the organic layer containing the product, wash with water/sodium chloride solution, and after drying, evaporate the solution to produce a dark oil which solidifies when triturated with hexane. The title product is obtained when the above solid material is crystallized from acetonitrile, m.p. 114.5—115.5°C.

Example 39

*Dibenzo[b,f]thiepin-3-carboxaldehyde 5-oxide*

Dissolve 512 mg. (2 mmole) of 3-hydroxymethyldibenzo[b,f]thiepin 5-oxide in 50 ml. of methylene chloride and add 648 mg. (3 mmole) of pyridinium chlorochromate. Stir at room temperature for 1.5 hours and then filter the mix through a bed of Florisil. Wash the Florisil with dichloromethane; and to the combined filtrate, add 4 g. of silica gel. Evaporate the suspension to dryness, and place the solid on top of a column of 50 g. of silica and elute the title compound with a 1:9 mixture of ethyl acetate and toluene. The compound has a m.p. of 188—190°C.

Example 40

*10,11-Dihydro-dibenzo[b,f]thiepin-3-carboxaldehyde*

Mix 4.74 g. (20 mmole) of 3-cyano-10,11-dihydrodibenzo[b,f]thiepin with 7.56 g. (40 mmole) anhydrous stannous chloride and 150 ml. ether saturated with anhydrous hydrogen chloride. Stir the mixture at 25°C. for approximately 50 hours to produce a crystalline solid suspended in the reaction mixture. Shake the crystalline solid with 200 ml. ice water, and extract with 150 ml. ethyl acetate using methanol to dissolve any residual lumpy yellow solid. Separate the ethyl acetate layer containing the product, and after washing with water/saturated sodium chloride solution and drying, the ethyl acetate is removed by evaporation to produce as a residue an amber-colored oil, which crystallizes overnight. Distill the crystalline residue under reduced pressure at 145°C. at 0.04 mm. The distilled product crystallizes, m.p. 55.5—56.5°C.

Example 41

*10,11-dihydro-dibenzo[b,f]thiepin-3-carboxaldehyde 5-oxide*

Mix 2.84 g. (11.83 mmole) of 10,11-dihydro-dibenzo[b,f]thiepin-3-carboxaldehyde with 90 ml. methylene chloride at 0°C. Add 2.3 g. (11.33 mmoles) of 85% m-chloroperbenzoic acid, followed by the addition of 100 ml. methylene chloride. Stir for 2 hours, and then add 4.6 g. calcium hydroxide to

the resulting clear solution at room temperature. After stirring the suspension for 15 minutes, filter to remove the inorganic solid, and evaporate the filtrate containing the dissolved product to dryness, producing as a residue a dry solid comprising the title product. Purify by recrystallization from acetonitrile, m.p. 158.5—160°C.

Example 42

*10,11-Dihydro-dibenzo[b,f]thiepin-3-carboxaldehyde 5,5-dioxide*

Repeat the procedure of Example 39, substituting an equivalent amount of 10,11-dihydro-3-hydroxymethyldibenzo[b,f]thiepin 5,5-dioxide in place of 3-hydroxymethyldibenzo[b,f]thiepin 5-oxide to obtain the title compound, m.p., 135—136°C.

Example 43

*8-Fluoro-dibenzo[b,f]thiepin-3-carboxaldehyde*

Dissolve 568 mg. (2.2 mmole) of 2-fluoro-7-hydroxymethyldibenzo[b,f]thiepin in 30 ml. of methylene chloride and add 647 mg. of 98% pyridinium chlorochromate. Stir the mixture and follow the reaction by thin layer chromatography (Merck silica gel F254 with 5% ethyl acetate in toluene). When complete, filter the mixture through a bed of Florisil, and wash through with methylene chloride. Evaporate the filtrate to obtain a brown solid (600 mg.). Purify the crude product by chromatography on silica gel using toluene as solvent to obtain the title compound as a yellow solid, m.p., 137—139°C.

Example 44

*8-Fluoro-dibenzo[b,f]thiepin-3-carboxaldehyde 5-oxide*

Repeat the procedure of Example 39, substituting an equivalent amount of 3-hydroxymethyl-8-fluorodibenzo[b,f]thiepin 5-oxide in place of the 3-hydroxymethyldibenzo[b,f]thiepin 5-oxide to obtain the title compound, m.p., 212—214°C.

Example 45

*8-Fluoro-dibenzo[b,f]thiepin-3-carboxaldehyde 5,5-dioxide*

Repeat the procedure of Example 39, substituting an equivalent amount of 3-hydroxymethyl-8-fluorodibenzo[b,f]thiepin 5,5-dioxide in place of 3-hydroxymethyldibenzo[b,f]thiepin 5-oxide to obtain the title compound, m.p., 200—202°C.

Example 46

*8-Fluoro-10,11-dihydrodibenzo[b,f]thiepin-3-carboxaldehyde*

Proceeding as in Example 39, but starting with 0.5 g. (1.9 mmole) of 8-fluoro-3-hydroxymethyl-10,11-dihydrodibenzo[b,f]thiepin, there is obtained 0.42 g. (84%) of 8-fluoro-3-formyl-10,11-dihydrodibenzo[b,f]thiepin, m.p., 78—79°C.

Example 47

*8-Fluoro-10,11-dihydrodibenzo[b,f]thiepin-3-carboxaldehyde 5-oxide*

Proceeding as in Example 39, but starting with 0.5 g. (1.8 mmole) of 8-fluoro-3-hydroxymethyl-10,11-dihydrodibenzo[b,f]thiepin-5-oxide, there is obtained 0.38 g. (77%) of 8-fluoro-3-formyl-10,11-dihydrodibenzo[b,f]thiepin 5-oxide, m.p., 188—189°C.

Example 48

*8-Fluoro-10,11-dihydrodibenzo[b,f]thiepin-3-carboxaldehyde 5,5-dioxide*

Proceeding as in Example 39, but starting with 0.5 g. (1.7 mmole) of 8-fluoro-3-hydroxymethyl-10,11-dihydrodibenzo[b,f]thiepin 5,5-dioxide, there is obtained 0.43 g. (87%) of 8-fluoro-3-formyl-10,11-dihydrodibenzo[b,f]thiepin 5,5-dioxide, m.p., 155—156°C.

Example 49

*8-Aminodibenzo[b,f]thiepin-3-carboxaldehyde 5,5-dioxide*

Repeat the procedure of Example 38, substituting an equivalent quantity of 8-amino-3-cyanodibenzo[b,f]thiepin 5,5-dioxide in place of 3-cyanodibenzo[b,f]thiepin 5,5-dioxide to obtain the title product.

Example 50

*8-Chlorodibenzo[b,f]thiepin-3-carboxaldehyde*

Repeat the procedure of Example 38, substituting an equivalent quantity of 8-chloro-3-cyanodibenzo[b,f]thiepin for the 3-cyano-dibenzo[b,f]thiepin to obtain the title product.

Example 51

*8-Chlorodibenzo[b,f]thiepin-3-carboxaldehyde 5-oxide and 5,5-dioxide*

Repeat the procedure of Example 41, substituting an equivalent quantity of 8-chlorodibenzo[b,f]thiepin-3-carboxaldehyde in place of the 10,11-dihydrodibenzo[b,f]thiepin-3-carboxaldehyde to obtain

the title 5-oxide compound.

Repeat the above procedure using twice the equivalent amount of m-chloroperbenzoic acid to obtain the corresponding 5,5-dioxide.

Example 52

*8-Hydroxydibenzo[b,f]thiepin-3-carboxaldehyde*

Repeat the procedure of Example 37, substituting an equivalent quantity of 3-cyano-8-hydroxydibenzo[b,f]thiepin in place of the 3-cyano-dibenzo[b,f]thiepin 5,5-dioxide to obtain the title compound.

Example 53

*8-Hydroxydibenzo[b,f]thiepin-3-carboxaldehyde 5,5-dioxide*

Repeat the procedure of Example 37 using an equivalent amount of 3-cyano-8-hydroxydibenzo[b,f]thiepin 5,5-dioxide in place of 3-cyano-dibenzo[b,f]thiepin 5,5-dioxide to obtain the title compound.

Example 54

*8-Methylthiodibenzo[b,f]thiepin-3-carboxaldehyde*

Repeat the procedure of Example 38, substituting 3-cyano-8-methylthio-dibenzo[b,f]thiepin in place of 3-cyano-dibenzo[b,f]thiepin to obtain the title product.

Example 55

*8-Methylsulfinyldibenzo[b,f]thiepin-3-carboxaldehyde*

Repeat the procedure of Example 41 using an equivalent amount of 8-methylthiodibenzo[b,f]-thiepin-3-carboxaldehyde in place of 10,11-dihydrodibenzo[b,f]thiepin-3-carboxaldehyde to obtain the title product.

Example 56

*8-Methylthiodibenzo[b,f]thiepin-3-carboxaldehyde 5-oxide*

Dissolve 8-methylthiodibenzo[b,f]thiepin-3-carboxaldehyde (5 mmole) in methylene chloride (100 ml.); and while cooling the solution in an ice-bath, add a 1 M solution of 90% nitric acid in methylene chloride (5 ml.). Monitor the reaction mixture by tlc after 30 minutes, and add the nitric acid solution in small increments until starting material has disappeared. Treat the reaction mixture with sodium bicarbonate solution to remove acidic substances. Dry the organic solution, evaporate to dryness, and purify the title product by column chromatography on silica gel using 1:4 ethyl acetate/toluene for elution.

Example 57

*8-Methylsulfinyldibenzo[b,f]thiepin-3-carboxaldehyde 5-oxide*

Repeat the procedure of Example 56 using 3 to 5 equivalents of 1 M nitric acid in methylene chloride in place of slightly more than one equivalent to obtain the title compound.

Example 58

*8-Methylthiodibenzo[b,f]thiepin-3-carboxaldehyde 5,5-dioxide*

React 8-methylthiodibenzo[b,f]thiepin-3-carboxylic acid 5,5-dioxide with excess thionyl chloride under reflux until conversion to the acid chloride is complete. Distill off excess thionyl chloride under vacuum and remove the last traces by adding benzene to the residue and evaporating under vacuum.

Dissolve the crude 8-methylthiodibenzo[b,f]-thiepin-3-carboxylic acid chloride 5,5-dioxide in glyme, and with stirring under dry nitrogen, add a filtered solution of 1.1 equivalents of lithium tri-tertiary butoxy-aluminum hydride in glyme dropwise with cooling (ice-bath). Stir the mixture for 1 hour after the addition is complete and pour into ice-cold dilute hydrochloric acid. Isolate the crude title compound by extraction with chloroform and purify the compound by column chromatography on silica gel using toluene as the solvent.

Example 59

*8-Methylsulfinyldibenzo[b,f]thiepin-3-carboxaldehyde 5,5-dioxide*

Repeat the procedure of Example 41, substituting an equivalent quantity of 8-methylthiodibenzo[b,f]thiepin-3-carboxaldehyde 5,5-dioxide in place of 10,11-dihydrodibenzo[b,f]thiepin-3-carboxaldehyde to obtain the title product.

Example 60

*8-Methylsulfonyldibenzo[b,f]thiepin-3-carboxaldehyde*

Repeat the procedure of Example 58, substituting 8-methylsulfonyldibenzo[b,f]thiepin-3-carboxylic acid in place of 8-methylthiodibenzo[b,f]thiepin-3-carboxylic acid 5,5-dioxide to obtain the title product.

Example 61

*8-Nitrodibenzo[b,f]thiepin-3-carboxaldehyde*

Heat a mixture of 8-nitrodibenzo[b,f]thiepin-3-carboxylic acid and excess thionyl chloride under reflux until conversion to the acid chloride is complete. Evaporate excess thionyl chloride under vacuum and remove traces of thionyl chloride from the product by treating the residue with benzene and evaporating again under vacuum.

Stir a mixture of the crude 8-nitrodibenzo[b,f]-thiepin-3-carboxylic acid chloride in dry tetrahydrofuran at —78°C. under nitrogen, and add dropwise a filtered solution of 1.1 equivalents of lithium tri-tertiarybutoxy-aluminum hydride in tetrahydrofuran. Stir the mixture for 30 minutes at —78°C. and allow to warm to ambient temperature. Add the mixture to ice-cold dilute hydrochloric acid, and isolate the product by extraction with chloroform. Purify the title compound by chromatography on silica gel using a mixture of ethyl acetate and toluene for elution.

Example 62

*8-Nitrodibenzo[b,f]thiepin-3-carboxaldehyde 5-oxide and 5,5-dioxide*

Repeat the procedure of Example 41, substituting 8-nitrodibenzo[b,f]thiepin-3-carboxaldehyde in place of 10,11-dihydrodibenzo[b,f]thiepin-3-carboxaldehyde to obtain the title 5-oxide compound.

Repeat the above procedure using twice the equivalent amount of m-chloroperbenzoic acid to obtain the corresponding 5,5-dioxide.

Example 63

*8-Methoxydibenzo[b,f]thiepin-3-carboxaldehyde*

Repeat the procedure of Example 39, substituting an equivalent quantity of 3-hydroxymethyl-8-methoxydibenzo[b,f]thiepin for the 3-hydroxymethyldibenzo[b,f]thiepin to obtain the title product.

Example 64

*8-Methoxydibenzo[b,f]thiepin-3-carboxaldehyde 5-oxide*

Repeat the procedure of Example 41, substituting an equivalent quantity of 8-methoxydibenzo[b,f]thiepin-3-carboxaldehyde in place of the 10,11-dihydrodibenzo[b,f]thiepin-3-carboxaldehyde to obtain the title 5-oxide compound.

Repeat the above procedure using twice the equivalent amount of m-chloroperbenzoic acid to obtain the corresponding 5,5-dioxide.

The compounds of formula I and IA wherein Z is defined as sulfinyl are capable of existing as optical isomers because of the tetrahedral structure of the sulfoxides substituent. Thus, the sulfoxide disclosed in the Examples are racemic mixtures of D and L isomers which may be resolved by known procedures into their enantiomers. Each of the enantiomorphic isomers may exhibit variation in biological potency. Thus, the compounds of Examples 2, 5, 8, 11, 23, 32, 35, 39, 41, 44, 47, 56, 57 and the 5-oxide compounds prepared in 51, 61 and 63 may be resolved by conventional procedures into their D and L enantiomorphs.

**Claims**

1. A compound of formula Ia

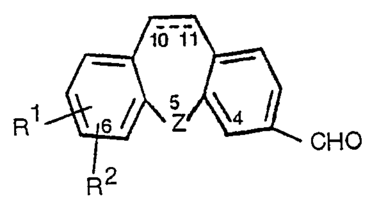

Ia

or Ib

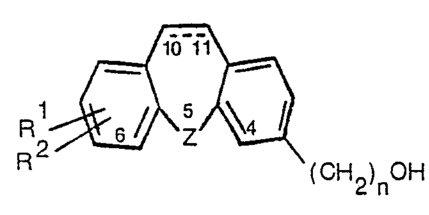

Ib

wherein
Z is thio, sulfinyl, or sulfonyl,
$R^1$ and $R^2$ are independently from each other hydrogen, halogen, amino, alkylamino having 1—4 carbon atoms in the alkyl group, dialkylamino having 1—4 carbon atoms in each of the alkyl groups, alkyl having 1—4 carbon atoms, $C_1$—$C_4$-hydroxyalkyl, phenyl-$C_1$—$C_4$-alkyl wherein the phenyl group is optionally substituted by halogen, an amino, hydroxy or $C_1$—$C_4$-alkyl group alkanoyl having 1—4 carbon atoms, hydroxy, thiol, alkoxy having 1—4 carbon atoms, alkylthio, alkylsulfinyl or alkylsulfonyl, wherein in each of said sulphur containing groups the alkyl group has 1—4 carbon atoms, trifluoromethyl, trifluoromethylthio, cyano or nitro
n is an integer of from 1—4 and
the dotted line indicates either an olefinic bond or saturation at the 10-, 11-position.

2. A compound of formula Ia or Ib according to patent claim 1, wherein Z is thio.

3. A compound of formula Ib according to claim 1, wherein a group of formula $(CH_2)_n$-OH in the 3-position is a hydroxy methyl group.

4. A compound of formula Ia or Ib according to one of the patent claims 1—3 wherein at least one of the radicals $R_1$ or $R_2$ is chloro, bromo, fluoro, iodo, hydroxy, methoxy, methylthio, methylsulfinyl, methylsulfonyl, nitro or hydrogen.

5. A process for preparing a compound of formula Ia according to claim 1

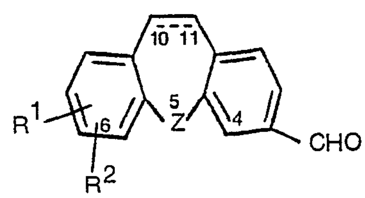

Ia

wherein
Z is thio, sulfinyl, or sulfonyl,
$R^1$ and $R^2$ are independently from each other hydrogen, halogen, amino, alkylamino having 1—4 carbon atoms in the alkyl group, dialkylamino having 1—4 carbon atoms in each of the alkyl groups, alkyl having 1—4 carbon atoms $C_1$—$C_4$-hydroxyalkyl, pheny-$C_1$—$C_4$-alkyl wherein the phenyl group is optionally substituted by halogen, an amino, hydro Y or $C_1$—$C_4$-alkyl group, alkanoyl having 1—4 carbon atoms, hydroxy, thio, alkoxy having 1—4 carbon atoms, alkylthio, alkylsulfinyl or alkylsulfonyl, wherein in each of said sulphur containing groups the alkyl group has 1—4 carbon atoms, trifluoromethyl, trifluoromethylthio, cyano or nitro and the dotted line indicates either an olefinic bond or saturation at the 10-, 11-position,
characterized in that a corresponding 3-cyano compound having the formula

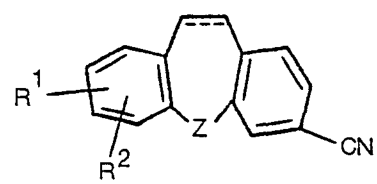

wherein $R^1$, $R^2$, Z and the dotted line have the same meaning as in formula Ia is reduced and hydrolized.

6. Process according to patent claim 5 characterized in that the reduction and hydrolization of the 3-cyano-compound is performed by either

a) reacting it with anhydrous stannous chloride in a dry inert solvent forming the corresponding aldiminium stannic chloride complex which is then hydrolized to form the corresponding 3-carboxaldehyde or
b) the cyano compound is treated with Raney alloy in aqueous formic acid or
c) 3-cyano compound is submitted to a selective reduction with sodium hypophosphite and Raney nickel in the presence of an aqueous acid.

7. Process for the preparation of a compound of formula Ib according to claim 1

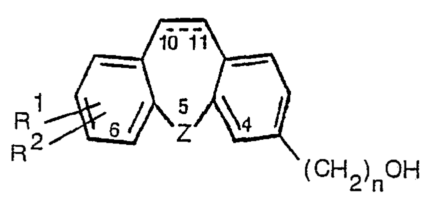

Ib

or Ia

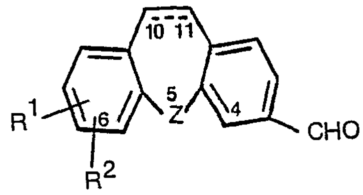

Ia

wherein
Z is thio, sulfinyl, or sulfonyl,
$R^1$ and $R^2$ are independently from each other hydrogen, halogen, amino, alkylamino having 1—4 carbon atoms in the alkyl group, dialkylamino having 1—4 carbon atoms in each of the alkyl groups, alkyl having 1—4 carbon atoms, $C_1$—$C_4$-hydroxyalkyl, phenyl-$C_1$—$C_4$-alkyl wherein the phenyl group is optionally substituted by halogen, an amino, hydroxy or $C_1$—$C_4$-alkyl group, alkanoyl having 1—4 carbon atoms, hydroxy, thiol, alkoxy having 1—4 carbon atoms, alkylthio, alkylsulfinyl or alkylsulfonyl, wherein in each of said sulphur containing groups the alkyl group has 1—4 carbon atoms, trifluoromethyl, trifluoromethylthio, cyano or nitro
n is an integer of from 1—4 and
the dotted line indicates either an olefinic bond or saturation at the 10-, 11-position,
characterized in that a carboxylic acid of formula

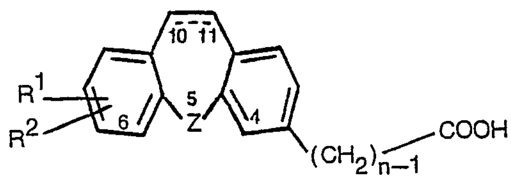

wherein
$R^1$, $R^2$, Z and n are as defined in formula Ib, or a lower alkyl ester thereof is reduced to form the corresponding hydroxyalkyl substituted compound of formula Ib, which compound of formula Ib is isolated and if in formula Ib n is 1, the 3-hydroxymethyl compound is optionally oxidized yielding the aldehyde of formula Ia.

8. Process according to patent claim 7 characterized in that the reduction reaction of the carboxylic acid or the lower alkyl ester thereof is performed using borane or an alkali metal aluminum hydride, preferably lithium aluminum hydride, as reducing agent, preferably in an inert solvent.

9. Process according to patent claim 7 characterized in that a compound of formula Ib wherein n is 1 is prepared which is thereafter oxidized by means of pyridinium chlorochromate in an inert solvent yielding the corresponding aldehyde of formula Ia.

10. A composition for treating undesirable contractile activity of prostaglandins characterized in that it comprises as active ingredient a compound of formula Ia

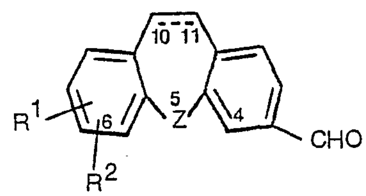

Ia

or Ib

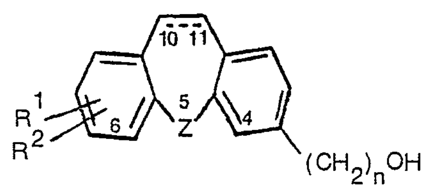

Ib

wherein
Z, $R^1$, $R^2$, n and the dotted line have the same meaning as in claim 1.

**Revendications**

1. Composé de formule Ia

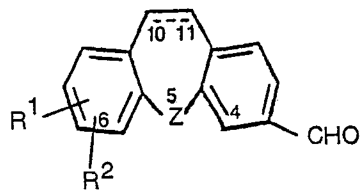

Ia

ou Ib

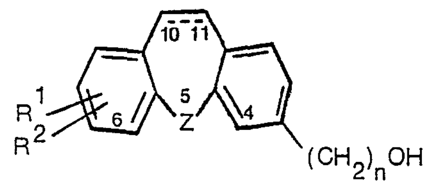

Ib

où
Z est un thio, un sulfinyle ou un sulfonyle,
$R^1$ et $R^2$ représentent indépendamment l'un de l'autre un hydrogène, un halogène, un amino, un alcoylamino en $C_1$ à $C_4$ dans le groupe alcoyle, un dialcoylamino en $C_1$ à $C_4$ dans chacun des groupes alcoyle, un alcoyle en $C_1$ à $C_4$, un hydroxyalcoyle en $C_1$ à $C_4$, un phényl-alcoyle en $C_1$ à $C_4$ où le groupe phényle est éventuellement substitué par un halogène, un amino, un hydroxy ou un groupe alcoyle en $C_1$ à $C_4$, un alcanoyle en $C_1$ à $C_4$, un hydroxy, un thiol, un alcoxy en $C_1$ à $C_4$, un alcoylthio, un alcoylsulfinyle ou un alcoylsulfonyle, où dans chacun desdits groupes contenant un soufre, le groupe alcoyle est en $C_1$ à $C_4$, un trifluorométhyle, un trifluorométhylthio, un cyano ou un nitro, n est un nombre entier allant de 1 à 4 et la ligne pointillée indique, soit une liaison oléfinique, soit une saturation en position 10,11.

2. Composé de formule Ia ou Ib selon la rev. 1 où Z est un thio.

3. Composé de formule Ib selon la rev. 1 où un groupe de formule $(CH_2)_n$-OH en position 3 est un groupe hydroxyméthyle.

4. Composé de formule Ia ou Ib selon l'une des rev. 1—3 où au moins l'un des radicaux $R_1$ ou $R_2$ est un chloro, un bromo, un fluoro, un iodo, un hydroxy, un méthoxy, un méthylthio, un méthylsulfinyle, un méthylsulfonyle, un nitro ou un hydrogène.

5. Procédé de préparation d'un composé de formule Ia selon la rev. 1

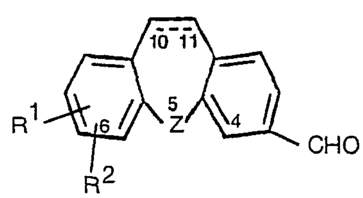

Ia

où
Z est un thio, un sulfinyle ou un sulfonyle,
$R^1$ et $R^2$ représentent indépendamment l'un de l'autre un hydrogène, un halogène, un amino, un alcoylamino en $C_1$ à $C_4$ dans le groupe alcoyle, un dialcoylamino en $C_1$ à $C_4$ dans chacun des groupes alcoyle, un alcoyle en $C_1$ à $C_4$, un hydroxylalcoyle en $C_1$ à $C_4$, un phényl-alcoyle en $C_1$ à $C_4$ où le groupe phényle est éventuellement substitué par un halogène, un amino, un hydroxy ou un groupe alcoyle en $C_1$ à $C_4$, un alcanoyle en $C_1$ à $C_4$, un hydroxy, un thiol, un alcoxy en $C_1$ à $C_4$, un alcoylthio, un alcoylsulfinyle ou un alcoylsulfonyle, où dans chacun desdits groupes contenant un soufre le groupe alcoyle est en $C_1$ à $C_4$, un trifluorométhyle, un trifluorométhylthio, un cyano ou un nitro, et la ligne pointillée indique, soit une liaison oléfinique, soit une saturation en position 10,11, caractérisé en ce qu'on réduit et hydrolyse un composé 3-cyano correspondant de formule

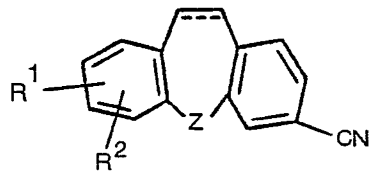

où $R^1$, $R^2$, Z et la ligne pointillée ont la même signification que dans la formule Ia.

6. Procédé selon la rev. 5, caractérisé en ce qu'on conduit la réduction et l'hydrolyse du composé 3-cyano

a) en la faisant réagir avec du chlorure stanneux anhydre dans un solvant inerte sec, en formant le complexe de chlorure stannique-aluminium correspondant que l'on hydrolyse alors pour former le 3-carboxaldéhyde correspondant ou

b) en traitant le composé cyano avec l'alliage de Raney dans l'acide formique aqueux ou

c) en soumettant le composé 3-cyano à une réduction sélective avec de l'hypophosphite de sodium et du nickel de Raney, en présence d'un acide aqueux.

7. Procédé de préparation d'un composé de formule Ib selon la rev. 1

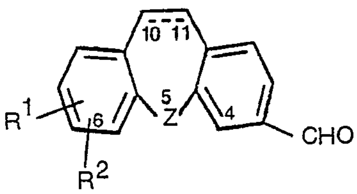

Ia

ou Ib

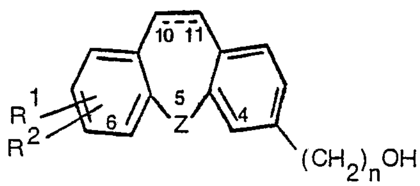

Ib

Z est un thio, un sulfinyle ou un sulfonyle,

$R^1$ et $R^2$ représentent indépendamment l'un de l'autre un hydrogène, un halogène, un amino, un alcoylamino en $C_1$ à $C_4$ dans le groupe alcoyle, un dialcoylamino en $C_1$ à $C_4$ dans chacun des groupes alcoyle, un alcoyle en $C_1$ à $C_4$, un hydroxyalcoyle en $C_1$ à $C_4$, un phényl-alcoyle en $C_1$ à $C_4$ où le groupe phényle est éventuellement substitué par un halogène, un amino, un hydroxy ou un groupe alcoyle en $C_1$ à $C_4$, un alcanoyle en $C_1$ à $C_4$, un hydroxy, un thiol, un alcoxy en $C_1$ à $C_4$, un alcoylthio, un alcoylsulfinyle ou un alcoylsulfonyle, où dans chacun desdits groupes contenant un soufre, le groupe alcoyle est en $C_1$ à $C_4$, un trifluorométhyle, un trifluorométhylthio, un cyano ou un nitro, n est un nombre entier allant de 1 à 4 et la ligne pointillée indique soit une liaison oléfinique, soit une saturation en position 10,11,

caractérisé en ce qu'on réduit un acide carboxylique de formule

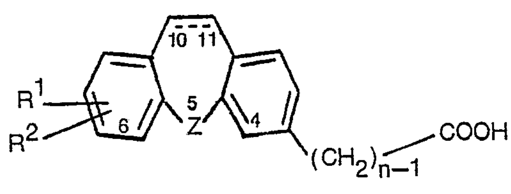

où

$R^1$, $R^2$, Z et n sont tels que définis dans la formule Ib, ou un de leurs alcoyle inférieur-esters, pour former le composé de formule Ib hydroxyalcoyl-substitué de formule Ib correspondant, lequel composé de formule Ib est isolé et si dans la formule Ib, n vaut 1, le composé 3-hydroxyméthyle est éventuellement oxydé, donnant l'aldéhyde de formule Ia.

8. Procédé selon la rev. 7 caractérisé en ce qu'on conduit la réaction de réduction de l'acide carboxylique ou de son alcoyle inférieur-ester en utilisant du borane ou un hydrure de métal alcalin-aluminium, de préférence l'hydrure de lithium-aluminium, comme agent réducteur, de préférence dans un solvant inerte.

9. Procédé selon la rev. 7 caractérisé en ce qu'on prépare un composé de formule Ib où n vaut 1, que l'on oxyde ensuite au moyen de chlorochromiate de pyridinium dans un solvant inerte, donnant l'aldéhyde de formule la correspondant.

10. Composition pour traiter l'activité contractile indésirable des prostaglandines, caractérisée en ce qu'elle comprend comme ingrédient actif un composé de formule la

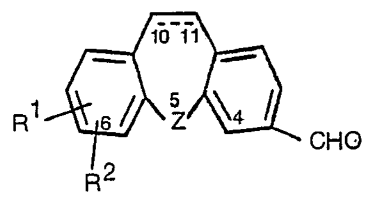

Ia

ou Ib

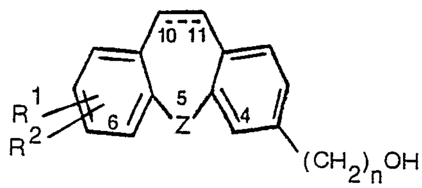

Ib

où
Z, $R^1$, $R^2$, n et la ligne pointillée ont la même signification que dans la rev. 1.

**Patentansprüche**

1. Verbindung mit der Formel Ia

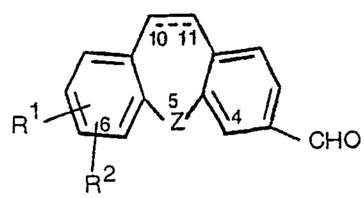

Ia

oder Ib

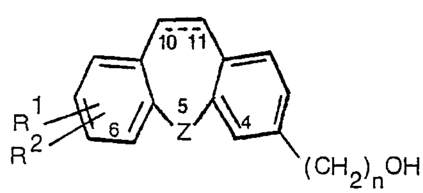

Ib

worin
Z Thio, Sulfinyl oder Sulfonyl ist,
$R^1$ und $R^2$ unabhängig voneinander Wasserstoff, Halogen, Amino, Alkylamino mit 1 bis 4 Kohlenstoffatomen in der Alkylgruppe, Dialkylamino mit 1 bis 4 Kohlenstoffatomen in jeder der Alkylgruppen, Alkyl mit 1 bis 4 Kohlenstoffatomen, $C_1$—$C_4$-Hydroxyalkyl, Phenyl-$C_1$—$C_4$-alkyl, worin die Phenylgruppe gegebenenfalls substituiert ist durch Halogen, eine Amino-, Hydroxy- oder $C_1$—$C_4$-Alkylgruppe, Alkanoyl mit 1 bis 4 Kohlenstoffatomen, Hydroxy, Thiol, Alkoxy mit 1 bis 4 Kohlenstoffatomen, Alkylthio, Alkylsulfinyl oder Alkylsulfonyl, worin in jeder der Schwefel enthaltenden Gruppen die Alkylgruppe 1 bis 4 Kohlenstoffatome aufweist, Trifluormethyl, Trifluormethylthio, Cyano oder Nitro, sind,
n eine ganze Zahl von 1 bis 4 ist und

die unterbrochene Linie entweder eine olefinische Bindung oder eine Sättigung in der 10-, 11-Stellung anzeigt.

2. Verbindung der Formel Ia oder Ib nach Patentanspruch 1, worin Z Thio ist.

3. Verbindung der Formel Ib nach Anspruch 1, worin eine Gruppe der Formel $(CH_2)_n$-OH in der 3-Stellung eine Hydroxymethylgruppe ist.

4. Verbindung der Formel Ia oder Ib gemäss einem der Patentansprüche 1 bis 3, worin mindestens einer der Reste $R_1$ oder $R_2$ Chlor, Brom, Fluor, Jod, Hydroxy, Methoxy, Methylthio, Methylsulfinyl, Methylsulfonyl, Nitro oder Wasserstoff ist.

5. Verfahren zur Herstellung einer Verbindung der Formel Ia gemäss Anspruch 1

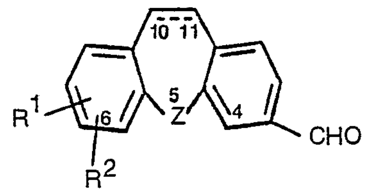

Ia

worin
Z Thio, Sulfinyl oder Sulfonyl ist,
$R^1$ und $R^2$ unabhängig voneinander Wasserstoff, Halogen, Amino, Alkylamino mit 1 bis 4 Kohlenstoffatomen in der Alkylgruppe, Dialkylamino mit 1 bis 4 Kohlenstoffatomen in jeder der Alkylgruppen, Alkyl mit 1 bis 4 Kohlenstoffatomen, $C_1$—$C_4$-Hydroxyalkyl, Phenyl-$C_1$—$C_4$-alkyl, worin die Phenylgruppe gegebenenfalls substituiert ist durch Halogen, eine Amino-, Hydroxy- oder $C_1$—$C_4$-Alkylgruppe, Alkanoyl mit 1 bis 4 Kohlenstoffatomen, Hydroxy, Thio, Alkoxy mit 1 bis 4 Kohlenstoffatomen, Alkylthio, Alkylsulfinyl oder Alkylsulfonyl, worin in jeder der Schwefel enthaltenden Gruppen die Alkylgruppe 1 bis 4 Kohlenstoffatome aufweist, Trifluormethyl, Trifluormethylthio, Cyano oder Nitro sind und die unterbrochene Linie entweder eine olefinische Bindung oder eine Sättigung in der 10-, 11-Stellung anzeigt, dadurch gekennzeichnet, dass eine entsprechende 3-Cyanoverbindung mit der Formel

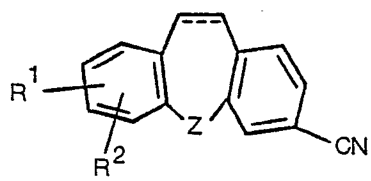

worin $R^1$, $R^2$, Z und die unterbrochene Linie die gleiche Bedeutung wie in der Formel Ia haben, reduziert und hydrolysiert wird.

6. Verfahren nach Patentanspruch 5, dadurch gekennzeichnet, dass die Reduktion und Hydrolyse der 3-Cyanoverbindung entweder durchgeführt wird durch

a) ihre Reaktion mit wasserfreiem Zinn-(II)-chlorid in einem trockenen inerten Lösungsmittel unter Bildung des entsprechenden Aldiminium-Zinn-(IV)-chlorid-Komplexes, der dann unter Bildung des entsprechenden 3-Carboxaldehyds hydrolysiert wird, oder

b) Behandeln der Cyanoverbindung mit Raneylegierung in wässriger Ameisensäure oder

c) Unterwerfen der 3-Cyanoverbindung einer selektiven Reduktion mit Natriumhypo phosphit und Raney-Nickel in Anwesenheit einer wässrigen Säure.

7. Verfahren zur Herstellung einer Verbindung der Formel Ib nach Anspruch 1

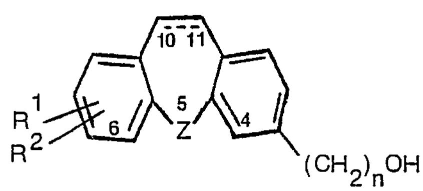

Ib

oder Ia

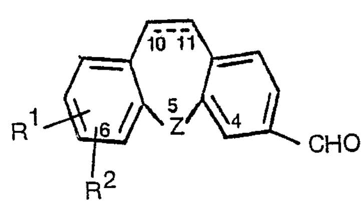

Ia

worin

Z Thio, Sulfinyl oder Sulfonyl ist,

$R^1$ und $R^2$ unabhängig voneinander Wasserstoff, Halogen, Amino, Alkylamino mit 1 bis 4 Kohlenstoffatomen in der Alkylgruppe, Dialkylamino mit 1 bis 4 Kohlenstoffatomen in jeder der Alkylgruppen, Alkyl mit 1 bis 4 Kohlenstoffatomen, $C_1$—$C_4$-Hydroxyalkyl, Phenyl -$C_1$—$C_4$-alkyl, worin die Phenylgruppe gegebenenfalls substituiert ist durch Halogen, eine Amino-, Hydroxy- oder $C_1$—$C_4$-Alkylgruppe, Alkanoyl mit 1 bis 4 Kohlenstoffatomen, Hydroxy, Thiol, Alkoxy mit 1 bis 4 Kohlenstoffatomen, Alkylthio, Alkylsulfinyl oder Alkylsulfonyl, worin in jeder der Schwefel enthaltenden Gruppen die Alkylgruppe 1 bis 4 Kohlenstoffatome aufweist, Trifluormethyl, Trifluormethylthio, Cyano oder Nitro, sind,

n eine ganze Zahl von 1 bis 4 ist und

die unterbrochene Linie entweder eine olefinische Bindung oder eine Sättigung in der 10-, 11-Stellung anzeigt, dadurch gekennzeichnet, dass eine Carbonsäure der Formel

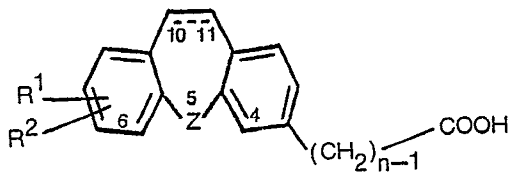

worin $R^1$, $R^2$, Z und n wie in der Formel Ib definiert sind, oder ein Niedrigalkylester davon reduziert wird unter Bildung der entsprechenden hydroxyalkylsubstituierten Verbindung der Formel Ib, wobei diese Verbindung der Formel Ib isoliert wird, und falls in der Formel Ib n 1 ist, die 3-Hydroxymethylverbindung gegebenenfalls oxidiert wird unter Bildung des Aldehyds der Formel Ia.

8. Verfahren nach Patentanspruch 7, dadurch gekennzeichnet, das die Reduktionsreaktion der Carbonsäure oder des Niedrigalkylesters davon durchgeführt wird unter Verwendung von Boran oder einem Alkalimetallaluminiumhydrid, vorzugsweise Lithiumaluminiumhydrid, als Reduktionsmittel, vorzugsweise in einem inerten Lösungsmittel.

9. Verfahren nach Patentanspruch 7, dadurch gekennzeichnet, dass eine Verbindung der Formel Ib, worin n 1 ist, hergestellt wird, die anschliessend mittels Pyridiniumchlorchromat in einem inerten Lösungsmittel oxidiert wird unter Bildung des entsprechenden Aldehyds der Formel Ia.

10. Zusammensetzung zur Behandlung unerwünschter kontrahierender Wirkung von Prostaglandinen, dadurch gekennzeichnet, dass sie als aktiven Bestandteil eine Verbindung der Formel Ia

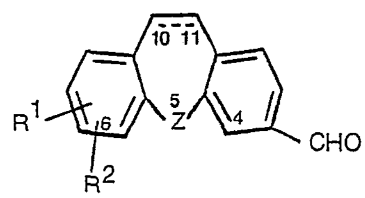

Ia

oder Ib

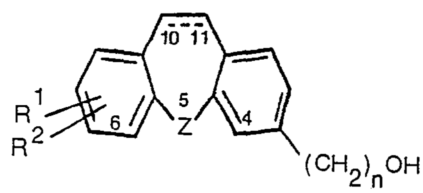

Ib

enthält, worin Z, $R^1$, $R^2$, n und die unterbrochene Linie die gleiche Bedeutung wie im Anspruch 1 haben.